# NEW EUROPEAN PATENT SPECIFICATION

(11) **EP 0 699 236 B2**
(45) Date of publication and mention of the opposition decision: **14.06.2006**
(45) Mention of the grant of the patent: 04.10.2000
(21) Application number: 94919120.9
(22) Date of filing: 18.05.1994
(51) Int. Cl.: C12N 15/12, C12P 21/02, C07K 14/475, C12P 21/08, A61K 38/18, G01N 33/68, C12N 1/21, C12N 5/10, A61K 39/00, C12Q 1/68

(54) **PURIFIED MAMMALIAN FLT3 LIGANDS AND AGONISTS AND ANTAGONISTS THEREOF**
GEREINIGTE FLT3 LIGANDEN VON SAEUGENTIEREN, AGONISTEN UND ANTAGONISTEN DAVON
LIGANDS PURIFIES DE FLT3 DE MAMMIFERES ET LEURS AGONISTES/ANTAGONISTES

(30) Priority: 19.05.1993 US 65231; 07.07.1993 US 89263; 16.07.1993 US 92549; 13.08.1993 US 106340; 24.08.1993 US 112391; 19.11.1993 US 155111; 03.12.1993 US 162413
(43) Date of publication of application: 06.03.1996
(60) Divisional application: 99116598.6
(73) Proprietor: SCHERING CORPORATION, Kenilworth New Jersey 07033 (US); INSTITUT NATIONAL DE LA SANTE ET DE LA RECHERCHE MEDICALE (INSERM), 75643 Paris Cédex 13 (FR)
(72) Inventor: HANNUM, Charles, H., Sunnyvale, CA 94087 (US); LEE, Frank, D., Palo Alto, CA 94301 (US); BIRNBAUM, Daniel, F-13009 Marseille (FR); CULPEPPER, Janice, A., Mountain View, CA 94040 (US)
(74) Representative: Adams, Harvey Vaughan John
(86) International application number: PCT/US1994/005150
(87) International publication number: WO 1994/026891

(56) References cited:
- EP-A- 0 354 808
- WO-A-92/17486
- NATURE, vol.368, no.6472, 14 April 1994 pages 643 - 648 HANNUM ET AL. 'Ligand for Flt3/Flk2 receptor tyrosine kinase regulates growth of haematopoietic stem cells and is encoded by variant RNAs'
- BLOOD, vol.83, no.10, 15 May 1994 pages 2795 - 2801 LYMAN ET AL. 'Cloning of the human homolog of the murine flt3 ligand : a growth factor for early haematopoietic progenitor cells'
- CELL, vol.75, no.6, 17 December 1993 pages 1157 - 1167 LYMAN ET AL. 'Molecular cloning of a ligand for the Flt3/Flk-2 tyrosine kinase receptor : a proliferative factor for primitive haematopoietic cells'
- ONCOGENE, vol.6, no.9, September 1991 pages 1641 - 1650 ROSNET ET AL. 'Murine Flt3, a gene encoding a novel tyrosine kinase receptor of the PDGFR/CSF1R family' cited in the application
- CELL, vol.65, 28 June 1991 pages 1143 - 1152 MATTHEWS ET AL. 'A receptor Tyrosine kinase specific to haematopoietic stem and progenitor cell-enriched populations' cited in the application

## Description

The present invention relates to compositions which function in controlling development and differentiation of mammalian cells, e.g., cells of a mammalian immune system. In particular, it relates to proteins and mimetics which regulate development, differentiation and function of various cell types, including hematopoietic cells.

### BACKGROUND OF THE INVENTION

Protein tyrosine kinases often play important roles in signal transduction leading to cellular proliferation. The large family of protein tyrosine kinases includes many growth factor receptors. See, e.g., Pawson, *et al.* (1990) *Trends in Genetics* 6:350-356. Growth factor receptors are important in control and regulation of cellular physiology and development Although these receptors have been found on various cell lineages, their specific roles in regulation of development of different cell lineages are generally poorly understood. Suggestions of a role for a protein tyrosine kinase in hematopoiesis have been largely based upon the identification of the c-kit receptor tyrosine kinase as the W locus, mutations in which affect erythroid and mast cell lineages. See, e.g.. Chabot, *et al.* (1988) *Nature* 335:88-89; and Geissler, *et al.* (1988) *Cell 55*:185-192.

Besides the gene product of the W locus, another protein tyrosine kinase had been isolated and characterized. See Matthews, *et al.* (1991) *Cell 65*:1143-1152; and Rosnet *et al*. (1991) *Oncogene 6*:1641-1650. This protein has been designated Fms-like tyrosine kinase 3 (Flt3) or Flk2. Although it has been localized to particular cell types, e.g., placenta, gonad, neural, and hematopoietic, its biological effects on cell differentiation and physiology have not been fully described.

Moreover, the receptor should mediate cellular signal transduction in response to a natural ligand. The nature of the ligand has yet to be identified, and its physiological effects and cell lineage specificity remain largely unlmown. The distribution of the receptor, however, suggests that the ligand has a role in regulating cell physiology and development in a multiplicity of cell lineages.

There thus is a need to know about the structural, biological, and physiological properties of the regulatory factors which naturally bind to the FH3 tyrosine kinase receptor.

WO 94/28391 (published after the filing date of the present application) discloses a polypeptide having the sequence Met Thr Val Leu Ala Pro Ala Trp Ser Pro Thr Thr Tyr Leu Leu Leu Leu Leu Leu Leu Ser Ser Gly Leu Ser Gly Thr Gln Asp Cys Ser Phe Gin His Ser Pro lie Ser Ser Asp Phe Ala Val Lys lie Arg Glu Leu Ser Asp Tyr Leu Leu Gln Asp Tyr Pro Val Thr Val Ala Ser Asn Leu Gln Asp Glu Glu Leu Cys Gly Gly Leu Trp Arg Leu Val Leu Ala Gin Arg Trp Met Gdu Arg Leu Lys Thr Val Ala Gly Ser Lys Met Gln Gly Leu Leu Glu Arg Val Asn Thr Glu Ile His Phe Val Thr Lys Cys Ala Phe Gln Pro Pro Pro Ser Cys Leu Arg Phe Val Gln Thr Asn Ile Ser Arg Leu Leu Gln Glu Thr Ser Glu Gln Leu Val Ala Leu Lys Pro Trp Ile Thr Arg Gln Asn Phe Ser Arg Cys Leu Glu Leu Gln Cys Gln Pro Asp Ser Ser Thr Leu Pro Pro Pro Trp Ser Pro Arg Pro Leu Glu Ala Thr Ala Pro Thr Ala Pro Gln Pro Pro Leu Leu Leu Leu Leu Leu Leu Pro Val Gly Leu Leu Leu Leu Ala Ala Ala Trp Cys Leu His Trp Gln Arg Thr Arg Arg Arg Thr Pro Arg Pro Gly Glu Gln Val Pro Pro Val Pro Ser Pro Gln Asp Leu Leu Leu Val Glu His.

### SUMMARY OF THE INVENTION

The present invention, which is based in part upon the discovery of natural ligands for the Flt3 tyrosine kinase receptor, fills the foregoing need. This invention relates to agonists and antagonists of the natural ligands, e.g., mutations (muteins) of the natural sequences, fusion proteins, chemical mimetics, antibodies, and other structural or functional analogs. It also provides isolated nucleic acids encoding proteins of the invention. Various uses of these different protein or nucleic acid compositions are also disclosed.

According to one aspect, the present invention provides an isolated myrine Fms-like tyrosine kinase 3 (Flt3) ligand which specifically binds to a Flt3 tyrosine kinase receptor which Fit3 ligand binds to an antibody produced against a murine Flt3 ligand characterized by:
(a) an apparent molecular weight of about 30 Kd in SDS-polyacrylamide gel electrophoresis;
(b) presence in a 60-85% saturated pellet during ammonium sulfate precipitation at 4°C;
(c) elution at between 900-750 mM (NH₄)₂SO₄ in 20 mM Tris buffer, pH 7.5, during gradient hydrophobic interaction chromatography using a phenyl-5PW column;
(d) elution at between 130-250 mM in a NaCl gradient in 20 mM Tris buffer, pH 7.5, during Mono Q column anion exchange chromatography;
(e) elution at between 440-540 mM in a NaCl gradient in 10mM citrate buffer, pH 3.0, during Mono S column cation exchange chromatography;
(f) an apparent molecular weight of 70 kD in SEPHACRYL® S200 gel filtration chromatography; and
(g) elution between 32-35% acetonitrile during reversed phase HPLC using a water acetonitrile gradient in 0.1 % TFA and a Poros R/H column.

In a second aspect, the invention further provides an isolated Fms-like tyrosine kinase 3 (Fit 3) ligand, comprising the amino acid sequence defined by SEQ ID No: 19.

Isolated nucleic acids encoding said Fit3 ligand comprising the amino acid sequence of SEQ ID No:19 form furth--er aspects of the present invention, especially an isolated nucleic acid encoding a said Fit3 ligand comprising the coding sequence of SEQ ID No: 19, as well as recombinant vectors comprising said nucleic acids and host cells harbouring said vectors, and methods for making said Fit3 ligand, comprising culturing the aforementioned host cells under conditions in which the nucleic acid is expressed.

Antibodies or binding fragments thereof which specifically binds to the above-defined murine a Flt3 ligand especially monoclonal antibodies, form further aspects of the invention.

Also provided are pharmaceutical compositions comprising a physiologically acceptable carrier and a Flt3 ligand comrising the amino acid sequence of SEQ 10 NO:19.

The murine Flt3 ligand may comprise at least one murine polypeptide sequence shown in Table 1. They further may exhibit a post-translational modification pattern distinct from natural Flt3 ligand, including at least one of the features disclosed in Table 2, or they may induce Flt3 receptors to self-phosphorylate. The invention further relates to a composition comprising such a ligand and a pharmaceutically acceptable carrier

In antibody embodiments the antibody may be raised against a murine peptide sequence of Table 1; the antibody is a manoclorial antibody; or the antibody is labeled.

The invention also relates to methods of modulating physiology or development of a cell comprising contacting said cell with an agonist or antagonist of a Flt3 ligand For example, the antagonist might be an antibody against the above defined murine Fit3 ligand or the cell may be a hematopoietic cell, including a lymphoid cell; a placenta cell; a gonad cell; or a neural cell, including neuronal or non-neuronal cells.
It is to be understood when reading the following description that the scope of protection is defined by the appendant claims. In relation to claims reciting specific amino acid or nucleic acid sequences (or claims dependent thereon), it will be understood that exactly the amino acid sequence of SEQ ID NO:19, or a nucleic acid sequence encoding that amino acid sequence as appropriate is required

### DESCRIPTION OF THE INVENTION

All references cited herein are hereby incorporated in their entirety by reference.

### General

The present invention provides the amino acid sequence and DNA sequences encoding the ligands of the second aspect that exhibit properties of binding to a tyrosine kinase receptor protein. The ligands of the invention are designated Flt3 ligands, because they were initially characterized as proteins which bind to the Flt3 protein, a protein which exhibits structural characteristics of a tyrosine kinase type of receptor.

The natural ligand of the invention are capable of mediating various biochemical responses which should lead to biological or physiological responses in target cells. Initial studies had localized the protein to hematopoietic stem cells and primtive un committed progenitors. The best characterized embodiment was initially described in mouse, but a human ligand is also described herein. Additional sequences for proteins in other mammalian species, e.g., human, should also be available. The descriptions below are directed, for exemplary purposes, to a mouse Flt3 ligand, but are likewise applicable to related embodiments from other species.

Isolated mouse Flt3 protein was recently described as a protein which exhibits structural features of a receptor tyrosine kinase. The protein is localized in placenta, gonad, hematopoietic, and neural tissues, among others. See Matthews, *et al.* (1991) *Cell 65*:1143-1152; and Rosnet, *et al.* (1991) *Oncogene 6*:1641-1650. The Flt3 receptor mediates a biochemical response to binding of a heretofore unidentified ligand leading to signal transduction and cellular response.

In particular, the ligand has been isolated by pursuing a self-phasphorylatian assay, which likely reflects cross phosphorylation of dimerized receptor molecules. The ligand has been isolated and characterized as a protein which migrates on polyacrylamide gel electrophoresis with a mobility characteristic of a protein of about 30 kD, while other physical properties are described in Table 2.

The ligand for Flt3 should be present in the mentioned tissue types, and the interaction of the ligand with receptor should be important for mediating various aspects of cellular physiology or development The distribution of the Flt3 receptor protein in different tissues suggests that it and its ligand have functional roles outside the immune system, e.g., in developmental regulation in other cell types. See, e.g., Gilbert (1991) *Developmental Biology* (3d ed.), Sinauer Associates, Sunderland, MA; Browder, *et al.* (1991) *Developmental Biology* (3d ed.), Saunders, Philadelphia, PA.; Russo, *et al.* (1992) *Development The Molecular Genetic Approach.* Springer-Verlag, New York, N.Y; and Wilkins (1993) *Genetic Analysis of Animal Development* (2d ed.) Wiley-Liss, New York, N.Y

When highly purified native mouse Flt3 ligand was added with IL-3 to mouse Thy^{lo} Sca-1⁺ lin⁻ stem cells, colony numbers were significantly increased. These conditions produced multilineage colonies, but they did not contain the abundant erythroid cells characteristically found with c-kit ligand and IL-3. A modest co-stimulatory activity was evident in the presence of Flt3 ligand and IL-6. However, Flt3 ligand alone had no stimulatory activity on these cells even when used in combination with c-kit ligand.

When sorted human fetal liver progenitor cells were used, Flt3 ligand had a similar synergistic effect in combination with GM-CSF or IL-3. In this case, the co-stimulatory effects of Flt3 ligand were observed on both low proliferative potential colony-forming cells (LPP-CFC) as well as the more primitive high proliferative potential colony-forming cells (HPP-CFC). However, in contrast to c-Kit ligand, Flt3 ligand was less able to augment the growth of human fetal liver burst-forming units erythroid (BFU-E). Thus, Flt3 ligand enhances the response of stem and primitive progenitor cells to growth factors but in a manner distinct from c-kit ligand.

With respect to myeloid progenitors. Flt3 ligand has little effect alone on proliferation, but in combination with such factors as GM-CSF or IL-3, the ligand synergistically can promote the growth of both primitive and more mature myeloid precursors.

Flt3 ligand, in combination with IL-7 or IL-12, activates specific thymocyte subsets to proliferate, CD4⁺, CD8⁺, and CD4^{lo} thymocytes are some of the subsets responding to the cytokine combinations

Flt3 ligand was also tested on day 14 fetal thymocytes which are enriched for T cell precursors. Flt3 ligand in combination with IL-7 or IL-12 induced significant proliferation. IL-12 also induces proliferation of fetal thymocytes in combination with c-kit ligand. These results further support a role for Flt3 ligand in T cell development.

The proliferation of early B lineage cells, e.g., a pro B cell line or bone marrow cells enriched for pro B and pre B cells, is significantly enhanced in the presence of Flt3 ligand, particularly in combination with IL-7. Early developmental stage cell populations which give rise to committed B lineage cells also proliferate in response to Flt3 ligand, particularly in combination with other stromal cell factors.

identification of the ligand for Flt3 provides means to address some of the questions raised by these observations.

### Purified Flt3 Ligand

Mouse and human Flt3 ligand amino add sequences are shown in Table 1. These amino acid sequences, shown amino to carboxy terminus, are important in providing sequence information in the ligand allowing for distinguishing the protein from other proteins. Moreover, the peptide sequences allow preparation of peptides to generate antibodies to recognize such segments, and allow preparation of oligonucleotide probes, both of which are strategies for isolation, e.g., cloning, of genes encoding such sequences.

In particular, the MB8 isolate contains an insert of 29 amino acids which contain proteolytic processing sites which will allow the helical cytokine domain of the ligand to be cleaved from a membrane attachment Similarities have been observed with other cytokines. See, e.g., Bosenberg, *et at.* (1992) *Cell 71*:1157-1165; Huang, *et. al.* (1992) *Molecular Biology of the Cell* 3:349-362; and Pandiella, *et al.* (1992) *J. Biol. Chem. 267*:24028-24033. This Will avoid certain problems of working with, or administering, a cell bound protein, and provides insight into possible mechanisms of cellular specificity.

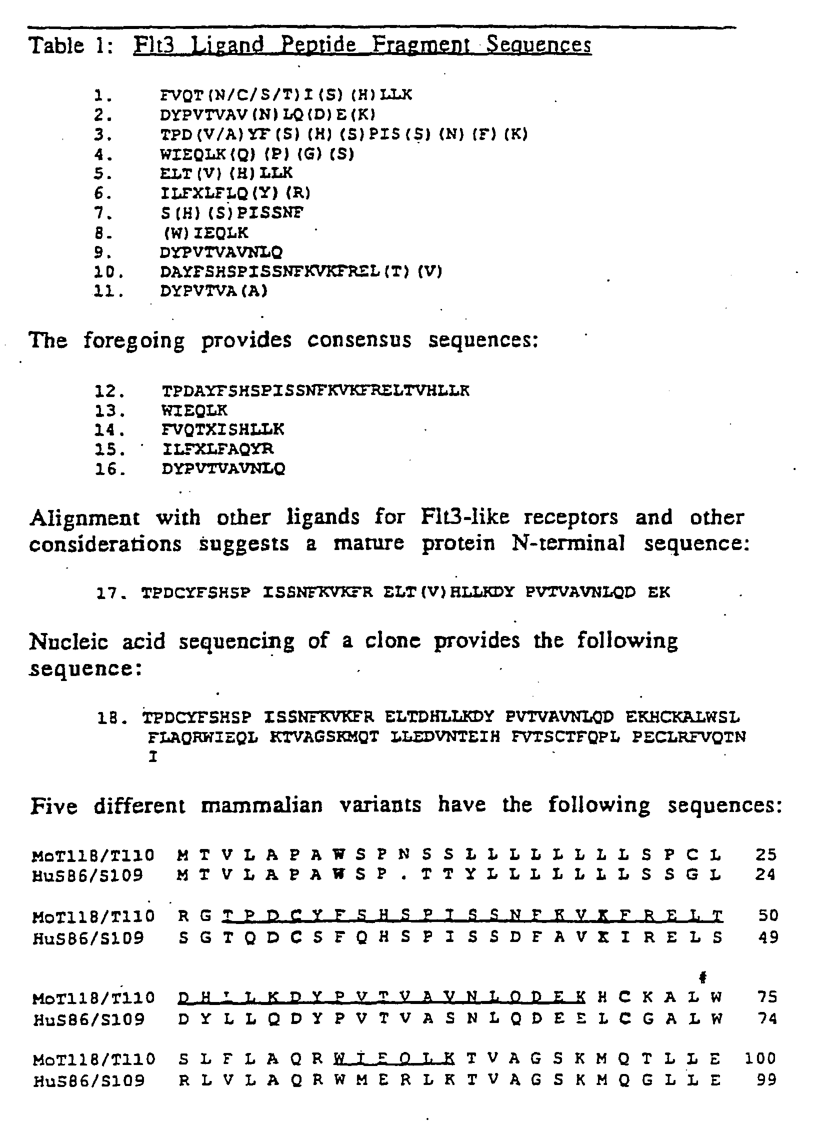

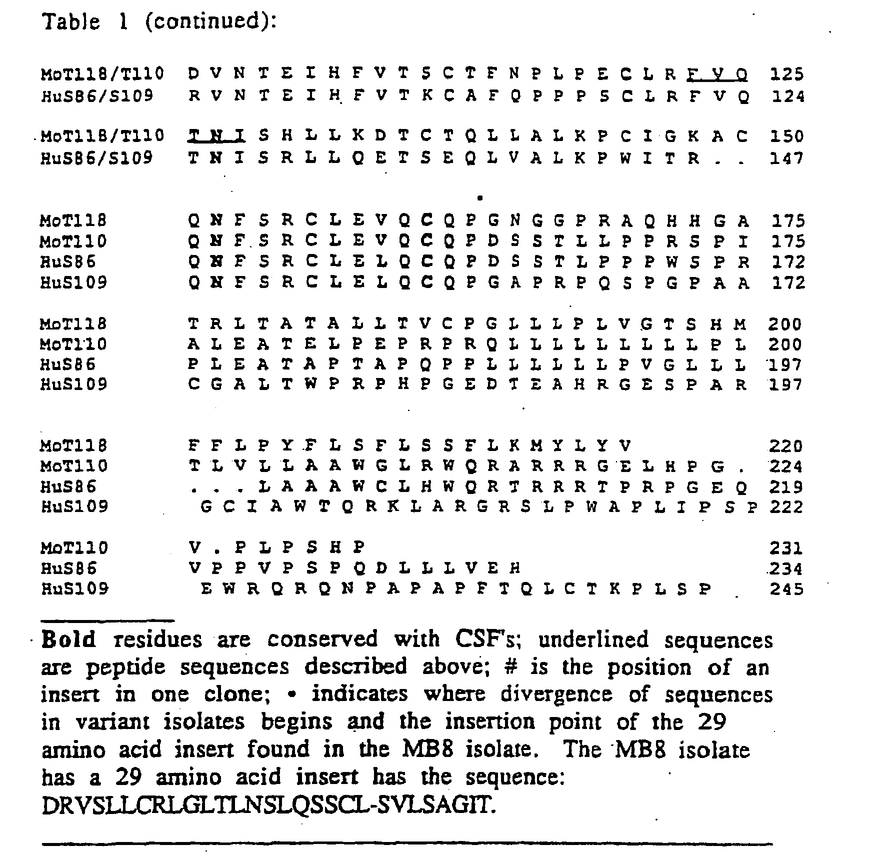

The sequences shown in Table 1 are also defined in the Sequence Listing, wherein the sequences of peptides 1-18 are defined by SEQ ID NOs: 1-18, respectively. Some amino acid residues in the sequences are indicated as Xaa, where there was some uncertainty in the sequence determinations.

Other sequences shown in Table 1 are defined in the Sequence Listing as follows:

| Peptide | SEQ ID NO: |
|---|---|
| HuS86/S109 (to S86 C-terminus) | 19 |
| HuS109 (C-terminal region) | 20 |
| MoT118/T110 (to T110 C-terminus) | 21 |
| MoT118 B (C-terminal region) | 22 |
| MB8 Isolate | 23 |

The nucleotide sequence for the C-terminal region of MoT118 is defined by SEQ ID NO: 24.

**Table 2 Physical Properties of Mouse Flt3 Ligand**

| |
|---|
| (1) SDS-Polyacrylamide gel electrophoresis: reduced migration approximately 30 Kd; seemingly a glycoprotein. |
| (2) Ammonium Sulfate precipitation (at 4° C): activity found in 60-85% saturated (NH₄)₂SO₄ pellet. |
| (3) Hydrophobic Interaction Chromatography [(NH₄)₂SO₄ gradient in 20 mM Tris, pH 7.5 on a Phenyl-5PW column]: activity eluted between 900-750 mM (NH4)₂SO₄. |
| (4) Anion Exchange Chromatography (NaCl gradient in 20 mM Tris, pH 7.5 on Mono Q column): activity eluted between 130-250 mM NaCl. |
| (5) Cation Exchange Chromatography (NaCl gradient in 10 mM citrate, pH 3.0 on Mono S column): the bulk of the activity eluted between 440-540 mM NaCl. |
| (6) Gel Filtration (SEPHACRYL® S200 column): the activity ran with an apparent molecular weight of 70 kD. |
| (7) Reversed Phase HPLC (water to acetonitrile gradient in 0.1% TFA on a Poros R/H colunm): the activity eluted between 32-35% acetonitrile. |

As used herein, the term "mouse Flt3 ligand" shall encompass, when used in a protein context, a protein having mouse amino acid sequences shown in Table 1. It also refers to a mouse derived polypeptide which exhibits similar biological function or interacts with Ftt3 ligand specific binding components. These binding components, e.g., antibodies, typically bind to a Flt3 ligand with high affinity, e.g., at least about 100 nM, usually better than about 30 nM, preferably better than about 10 nM, and more preferably at better than about 3 nM. Homologous proteins would be found in mammalian species other than mouse, e.g., rats. Non-mammalian species should also possess structurally or functionally related genes and proteins.

The term "poiypeptide" as used herein includes a significant fragment or segment, and encompasses a stretch of amino add residues of at least about 8 amino acids, generally at least 10 amino acids, more generally at least 12 amino acids, often at least 14 amino acids, more often at least 16 amino acids, typically at least 18 amino acids. more typically at least 20 amino acids, usually at least 22 amino acids, more usually at least 24 amino acids, preferably at least 26 amino acids, more preferably at least 28 amino acids, and, in particularly preferred embodiments, at least about 30 or more amino acids.

The term "binding composition'' refers to molecules that bind with specificity to Flt3 ligand, e.g., in a ligand-receptor type fashion, an antibody-antigen interaction, or compounds, e.g., proteins which specifically associate with Flt3 ligand, e.g., in a natural physiologically relevant protein-protein interaction, either covalent or non-covalent. The molecule may be a polymer, or chemical reagent No implication as to whether Fit3 ligand is either the ligand or the receptor of a ligand-receptor interaction is represented, other than the interaction exhibit similar specificity, e.g., specific affinity.

A functional analog may be a ligand with structural modifications, or may be a wholly unrelated molecule, e.g., which has a molecular shape which interacts with the appropriate ligand binding determinants. The ligands may serve as agonists or antagonists of the receptor, see, e.g., Goodman, *et al*. (eds.) (1990) *The Pharmacological Bases of Therapeutics* (8th ed.), Pergamon Press.

Substantially pure typically means that the protein is free from other contaminating proteins, nucleic acids, and other biologicals derived from the original source organism. Purity may be assayed by standard methods, and will ordinarily be at least about 40% pure, more ordinarily at least about 50% pure, generally at least about 60% pure, more generally at least about 70% pure, often at least about 75% pure, more often at least about 80% pure, typically at least about 85% pure, more typically at least about 90% pure, preferably at least about 95% pure, more preferably at least about 98% pure, and in most preferred embodiments, at least 99% pure.

Solubility of a polypeptide or fragment depends upon the environment and the polypeptide. Many parameters affect polypeptide solubility, including temperature, electrolyte environment, size and molecular characteristics of the polypeptide, and nature of the solvent. Typically, the temperature at which the polypeptide is used ranges from about 4° C to about 65° C. Usually the temperature at use is greater than about 18° C and more usually greater than about 22° C. For diagnostic purposes, the temperature will usually be about room temperature or warmer, but less than the denaturation temperature of components in the assay. For therapeutic purposes, the temperature will usually be body temperature, typically about 37° C for humans, though under certain situations the temperature may be raised or lowered *in situ* or *in vitro.*

The electrolytes will usually approximate *in situ* physiological conditions, but may be modified to higher or lower ionic strength where advantageous. The actual ions may be modified, e.g., to conform to standard buffers used in physiological or analytical contexts.

The size and structure of the polypeptide should generally be in a substantially stable state, and usually not in a denatured state. The polypeptide may be associated with other polypeptides in a quaternary structure, e.g., to confer solubility, or associated with lipids or detergents in a manner which approximates natural lipid bilayer interactions.

The solvent will usually be a biologically compatible buffer, of a type used for preservation of biological activities, and will usually approximate a physiological solvent. Usually the solvent will have a neutral pH, typically between about 5 and 10, and preferably about 7.5. On some occasions, a detergent will be added, typically a mild non-denaturing one, e.g., CHS or CHAPS, or a low enough concentration as to avoid significant disruption of structural or physiological properties of the ligand.

Solubility is reflected by sedimentation measured in Svedberg units, which are a measure of the sedimentation velocity of a molecule under particular conditions. The determination of the sedimentation velocity was classically performed in an analytical ultracentrifuge, but is typically now performed in a standard ultracentrifuge. See, Freifelder (1982) *Physical Biochemistry* (2d ed.), W.H. Freeman; and Cantor and Schimmel (1980) *Biophysical Chemistry,* parts 1-3, W.H. Freeman & Co., San Francisco. As a crude determination, a sample containing a putatively soluble polypeptide is spun in a standard full sized ultracentrifuge at about 50K rpm for about 10 minutes, and soluble molecules will remain in the supernatant. A soluble particle or polypeptide will typically be less than about 30S, more typically less than about 15S, usually less than about 10S, more usually less than about 6S, and, in particular embodiments, preferably less than about 4S, and more preferably less than about 3S.

Two specific biological activities of the Flt3 ligand are described below. The first is a ligand-dependent activity conferred on receptor-transformed or appropriate test cells. The second is a ligand-dependent autophosphorylation of receptor. These two biological activities have been utilized to isolate an appropriate ligand.

### Physical Variants

Also disclosed herein are proteins or peptides having substantial amino acid sequence homology with the amino acid sequence of the Flt3 ligand. The variants include species or allelic variants.

Amino acid sequence homology, or sequence identity, is determined by optimizing residue matches, if necessary, by introducing gaps as required. This changes when considering conservative substitutions as matches. Conservative substitutions typically include substitutions within the following groups: glycine, alanine; valine, isoleucine, leucine; aspartic acid, glutamic acid; asparagine, glutamine: serine, threonine; lysine, arginine; and phenylalanine, tyrosine. Homologous amino acid sequences are typically intended to include natural allelic and interspecies variations in each respective protein sequence. Typical homologous proteins or peptides will have from 25-100% homology (if gaps can be introduced), to 50-100% homology (if conservative substitutions are included) with the amino acid sequence of the Flt3 ligand. Homology measures will be at least about 35%, generally at least 40%, more generally at least 45%, often at least 50%, more often at least 55%, typically at least 60%, more typically at least 65%, usually at least 70%, more usually at least 75%, preferably at least 80%, and more preferably at least 80%, and in particularly preferred embodiments, at least 85% or more.

See also Needleham, *et al.* (1970) *J. Mol. Biol.* 48:443-453; Sankoff, *et al.* (1983) Chapter One in *Time Warps, String Edits, and Macromolecules: The Theory and Practice of Sequence Comparison* Addison-Wesley, Reading, MA; and software packages from lntelliGenetics, Mountain View, CA: and the University of Wisconsin Genetics Computer Group, Madison, WI.

The isolated Flt3 ligand DNA can be readily modified by nucleotide substitutions, nucleotide deletions, nucleotide insertions, and inversions of nucleotide stretches. These modifications result in novel DNA sequences which encode these antigens, their derivatives, or proteins having similar physiological, immunogenic, or antigenic activity. These modified sequences can be used to produce mutant antigens or to enhance expression. Enhanced expression may involve gene amplification, increased transcription, increased translation, and other mechanisms. Such mutant Flt3 ligand derivatives include predetermined or site-specific mutations of the respective protein or its fragments. "Mutant Flt3 ligand" encompasses a polypeptide otherwise falling within the homology definition of the mouse Flt3 ligand as set forth above, but having an amino acid sequence which differs from that of Flt3 ligand as found in nature, whether by way of deletion, substitution, or insertion.

In particular, "site specific mutant Flt3 ligand" generally includes proteins having significant homology with a ligand having sequences of Table 1, and as sharing various biological activities, e.g., antigenic or immunogenic, with those sequences, and in preferred embodiments contain most of the disclosed sequences.

Although site specific mutation sites are predetermined, mutants need not be site specific. Flt3 ligand mutagenesis can be conducted by making amino acid insertions or deletions. Substitutions, deletions, insertions, or any combinations may be generated to arrive at a final construct Insertions include amino- or carboxy- terminal fusions. Random mutagenesis can be conducted at a target codon and the expressed mutants can then be screened for the desired activity. Methods for making substitution mutations at predetermined sites in DNA having a known sequence are well known in the art, e.g., by M13 primer mutagenesis or polymerase chain reaction (PCR) techniques. See also Sambrook. *et al.* (1989) and Ausubel, *et al.* (1987 and Supplements).

The mutations in the DNA normally should not place coding sequences out of reading frames and preferably will not create complementary regions that could hybridize to produce secondary mRNA structure such as loops or hairpins.

The present invention also provides recombinant proteins, e.g., heterologous fusion proteins comprising the amino acid sequence of SEQ ID NO.19. A heterologous fusion protein is a fusion of proteins which are naturally not normally fused in the same manner. Thus, the fusion product of an immunoglobulin with a Flt3 ligand polypeptide is a continuous protein molecule having sequences fused in a typical peptide linkage, typically made as a single translation product and exhibiting properties derived from each source peptide. A similar concept applies to heterologous nucleic acid sequences.

In addition, new constructs may be made from combining similar functional domains from other proteins. For example, ligand-binding or other segments may be "swapped" between different new fusion polypeptides or fragments. See, e.g., Cunningham, *et al.* (1989) *Science 243*:1330-1336; and O'Dowd, *et al.* (1988) *J*. *Biol. Chem. 263*:15985-15992 Thus, new chimeric polypeptides exhibiting new combinations of specificities will result from the functional linkage of ligand-binding specificities and other functional domains.

The phosphoramidite method described by Beaucage and Carruthers (1981) *Tetra. Letts. 22*:1859-1862, will produce suitable synthetic DNA fragments. A double stranded fragment will often be obtained either by synthesizing the complementary stand and annealing the strand together under appropriate conditions or by adding the complementary strand using DNA polymerase with an appropriate primer sequence, e.g., PCR techniques.

### Functional Variants

The blocking of physiological response to FIt3 ligands may result from the inhibition of binding of the ligand to the FIt3 receptor, likely through competitive inhibition. Thus, *in vitro* assays will often use isolated protein, membranes from cells expressing a recombinant membrane associated Flt3 ligand, soluble fragments comprising receptor binding segments of these ligands, or fragments attached to solid phase substrates. These assays will also allow for the diagnostic determination of the effects of either binding segment mutations and modifications, or ligand mutations and modifications, e.g., ligand analogs.

This invention also contemplates the use of competitive drug screening assays, e.g., where neutralizing antibodies to antigen or receptor fragmerrts compete with a test compound for binding to the protein. In this manner, the antibodies can be used to detect the presence of any polypeptide which shares one or more antigenic binding sites of the ligand and can also be used to occupy binding sites on the protein that might otherwise interact with a receptor.

Additionally, neutralizing antibodies against Fit3 ligand and soluble fragments of the ligand which contain a high affinity receptor binding site, can be used to inhibit ligand function in tissues, e.g., tissues experiencing abnormal Physiology.

"Derivatives" of Fit3 ligand antigens include amino acid sequence mutants, glycosylation variants, and covatent or aggregate conjugates with other chemical moieties. Covalent derivatives can be prepared by linkage of functonalities to groups which are found in Fit3 ligand amino acid side chains or at the N- or C-termini, by means which are well known in the art. These derivatives can include, without limitation, aliphatic esters or amides of the carboxyl terminus, or of residues containing carboxyl side chains, O-acyl derivatives of hydroxyl group-containing residues, and N-acyl derivatives of the amino terminal amino acid or amino-group containing residues, e.g., lysine or arginine. Acyl groups are selected from the group of alkyl-moieties including C3 to C18 normal alkyl, thereby forming alkanoyl aroyl species. Covalent attachment to carrier proteins may be important when immunogenic moieties are haptens.

In particular, glycosylation alterations are included. e.g., made by modifying the glycosylation patterns of a polypeptide during its synthesis and processing, or in further processing steps. Particularly preferred means for accomplishing this are by exposing the polypeptide to glycosylating enzymes derived from cells which normally provide such processing, e.g., mammalian glycosylation enzymes. Deglycosylation enzymes are also contemplated. Also embraced are versions of the same primary amino acid sequence which have other minor modifications, including phosphorylated amino acid residues, e.g., phosphotyrosine, phosphoserine, or phosphothreonine.

A major group of derivatives are covalent conjugates of the Flt3 ligand or fragments thereof with other proteins or polypeptides. These derivatives can be synthesized in recombinant culture such as N- or C-terminal fusions or by the use of agents known in the art for their usefulness in cross-linking proteins through reactive side groups. Preferred ligand derivarization sites with cross-linking agents are at free amino groups, carbohydrate moieties, and cysteine residues.

Fusion polypeptides between Flt3 ligands and other homologous or heterologous proteins are also provided. Many growth factors and cytokines are homodimeric entities, and a repeat construct may have various advantages, including lessened susceptibility to proteolytic cleavage. Moreover, many receptors require dimerization to transduce a signal, and various dimeric ligands or domain repeats can be desirable. Homologous polypeptides may be fusions between different surface markers, resulting in, e.g., a hybrid protein exhibiting receptor binding specificity. Likewise, heterologous fusions may be constructed which would exhibit a combination of properties or activities of the derivative proteins.

Typical examples are fusions of a reporter polypeptide, e.g., luciferase, with a segment or domain of a ligand, e.g., a receptor-binding segment, so that the presence or location of the fused ligand may be easily determined. See, e.g., Dull, *et al.*, U.S. Patent No. 4,859,609. Other gene fusion partners include bacterial β-galactosidase, trpE, Protein A, β-faciamase, alpha amylase, alcohol dehydrogenase, and yeast alpha mating factor. See, e.g., Godowsid, et *al.* (1988) *Science* 241 :812-816..

The phosphoramidite method described by Beaucage and Carruthers (1981) *Tetra. Letts. 22*:1859-1862, will produce suitable synthetic DNA fragments. A double stranded fragment will often be obtained either by synthesizing the complementary strand and annealing the strand together under appropriate conditions or by adding the complementary strand using DNA polymerase with an appropriate primer sequence.

Such polypeptides may also have amino acid residues which have been chemically modified by phosphorylation, sulfonation, biotinylation, or the addition or removal of other moieties, particularly those which have molecular shapes similar to phosphate groups. In some embodiments, the modifications will be useful labeling reagents, or serve as purification targets, e.g., affinity ligands.

Fusion proteins will typically be made by either recombinant nucleic acid methods or by synthetic polypeptide methods. Techniques for nucleic acid manipulation and expression are described generally, for example, in Sambrook, *et al.* (1989) *Molecular Cloning*: A Laboratory Manual (2d ed.), Vols. 1-3, Cold Spring Harbor Laboratory. Techniques for synthesis of polypeptides are described, for example, in Merrifield (1963) *J. Amer. Chem.* Soc. *85:*2149-2156; Merrifield (1986) *Science* 232: 341-347; and Atherton, *et al*. (1989) *Solid Phase Peptide Synthesis*: *A Practical* Approach, IRL Press, Oxford.

This invention also contemplates the use of derivatives of Fit3 ligands other than variations in amino acid sequence or glycosylation. Such derivatives may involve covalent or aggregative association with chemical moieties. These derivatives generally fall into the three classes: (1) salts, (2) side chain and terminal residue covalent modifications, and (3) adsorption complexes, for example with cell membranes. Such covalent or aggregative derivatives are useful as immunogens, as reagents in immunoassays, or in purification methods such as for affinity purification of ligands or other binding ligands. For example, a Flt3 ligand antigen can be immobilized by covalent bonding to a solid support such as cyanogen bromide-activated SEPHAROSE® , by methods which are well known in the art, or adsorbed onto polyolefin surfaces, with or without glutaraldehyde cross-linking, for use in the assay or purification of anti-Flt3 ligand antibodies or its receptor.

The Flt3 ligands can also be labeled with a detectable group, for example radioiodinated by the chloramine T procedure, covalently bound to rare earth chelates, or conjugated to another fluorescent moiety for use in diagnostic assays. Purification of Flt3 ligand may be effected by immobilized antibodies or receptor.

A solubilized Flt3 ligand or fragment as described herein can be used as an immunogen for the production of antisera or antibodies specific for the ligand or any fragments thereof. The purified ligands can be used to screen monoclonal antibodies or ligand-binding fragments prepared by immunization with various forms of impure preparations containing the protein. In particular, the term "antibodies" also encompasses antigen binding fragments of natural antibodies. Purified Flt3 ligands can also be used as a reagent to detect antibodies generated in response to the presence of elevated levels of the ligand or cell fragments containing the ligand, both of which may be diagnostic of an abnormal or specific physiological or disease condition.

Additionally, ligand fragments may also serve as immunogens to produce the antibodies of the present invention, as described immediately below. For example, antibodies may be raised against amino acid sequences encoded by nucleotide sequences shown in Table 1, or fragments of proteins containing them. In particular, this invention contemplates antibodies having binding affinity to or being raised against specific fragments which are predicted to lie outside of the lipid bilayer.

The present invention contemplates the isolation of additional closely related species variants. Southern and Northern blot analysis should establish that similar genetic entities exist in other mammals. It is likely that Flt3 ligands are widespread in species variants, e.g., rodents, lagomorphs, carnivores, artiodactyla, perissodactyla, and primates.

The invention also provides means to isolate a group of related antigens displaying both distinctness and similarities in structure, expression, and function. Elucidation of many of the physiological effects of the ligands will be greatly accelerated by the isolation and characterization of distinct species variants of the ligands. In particular, the present invention provides useful probes for identifying additional homologous genetic entities in different species.

The isolated genes will allow transformation of cells lacking expression of a corresponding Flt3 ligand, e.g., either species types or cells which lack corresponding ligands and exhibit negative background activity. Expression of transformed genes will allow isolation of antigenically pure cell lines, with defined or single specie variants. This approach will allow for more sensitive detection and discrimination of the physiological effects of any Flt3 receptor proteins. Subcellular fragments, e.g., cytoplasts or membrane fragments, can be isolated and used.

Dissection of critical structural elements which effect the various differentiation functions provided by ligands is possible using standard techniques of modern molecular biology, particularly in comparing members of the related class. See, e.g., the homolog-scanning mutagenesis technique described in Cunningham, *et al.* (1989) *Science 243*:1339-1336; and approaches used in O'Dowd, *et al.* (1988) *J. Biol. Chem. 263*:15985-15992; and Lechleiter, *et al.* (1990) *EMBO J. 9*:4381-4390.

In particular, receptor binding segments can be substituted between species variants to determine what Structural features are important in both receptor binding affinity and specificity, as well as signal transduction. An array of different ligand variants will be used to screen for ligands exhibiting combined properties of interaction with differerrt receptor species variants.

Intracellular functions would probably involve segments of the receptor which are normally accessible to the cytosol. However, ligand internalization may occur under certain circumstances, and interaction between intracellular components and "extracellular" segments may occur. The specific segments of interaction of Flt3 ligand with other intracellular components may be identified by mutagenesis or direct biochenucal means, e.g., cross-linking or affinity methods. Structural analysis by crystallographic or other physical methods will also be applicable. Further investigation of the mechanism of signal transduction will include study of associated components which may be isolatable by affinity methods or by genetic means, e.g., complementation analysis of mutants.

Further study of the expression and control of Flt3 ligand will be pursued. The controlling elements associated with the ligands may exhibit differential developmental, tissue specific, or other expression patterns. Upstream or downstream genetic regions, e.g., control elements, are of interest. In particular, developmental or physiological variants, e.g., multiple alternatively processed forms of ligand have been found. See, e.g., Table 3. Thus, differential splicing of message may lead to membrane bound forms, soluble forms, and modified versions of ligand.

Structural studies of the ligands will lead to design of new ligands, particularly analogs exhibiting agonist or antagonist properties on the receptor. This can be combined with previously described screening methods to isolate ligands exhibiting desired spectra of activities.

Expression in other cell types will often result in glycosylation differences in a particular ligand. Various species variants may exhibit distinct functions based upon structural differences other than amino acid sequence. Differential modifications may be responsible for differential function, and elucidation of the effects are now made possible.

Thus, the present invention provides important reagents related to a physiological ligand-receptor interaction.

### Antibodies

Antibodies can be raised to various Flt3 ligands, including species or allelic variants, and fragments thereof, both in their naturally occurring forms and in their recombinant forms. Additionally, antibodies can be raised to Flt3 ligands in either their active forms or in their inactive forms. Anti-idiotypic antibodies are also contemplated.

Antibodies, including binding fragments and single chain versions, against predetermined fragments of the ligands can be raised by immunization of animals with conjugates of the fragments with immunogenic proteins. Monoclonal antibodies are prepared from cells secreting the desired antibody. These antibodies can be screened for binding to normal or defective Flt3 ligands, or screened for agonistic or antagonistic activity, e.g., mediated through the receptor. These monoclonal antibodies will usually bind with at least a K_{D} of about 1 mM, more usually at least about 300 µM, typically at least about 10 µM, more typically at least about 30 µM, preferably at least about 10 µM, and more preferably at least about 3 µM or better.

The antibodies, including antigen binding fragments, of this invention can have significant diagnostic or therapeutic value. They can be potent antagonists that bind to the receptor and inhibit ligand binding or inhibit the ability of a ligand to elicit a biological response. They also can be useful as non-neutralizing antibodies and can be coupled to toxins or radionuclides so that when the antibody binds to ligand, a cell expressing it, e.g., on its surface, is kitted. Further, these antibodies can be conjugated to drugs or other therapeutic agents, either directly or indirectly by means of a linker, and may effect drug targeting.

The antibodies of this invention can also be useful in diagnostic applications. As capture or non-neutralizing antibodies, they can be screened for ability to bind to the ligands without inhibiting receptor binding. As neutralizing antibodies, they can be useful in competitive binding assays. They will also be useful in detecting or quantifying Flt3 ligand or its receptors.

Ligand fragments may be joined to other materials, particularly polypeptides, as fused or covalently joined polypeptides to be used as immunogens. A ligand and its fragments may be fused or covalently linked to a variety of immunogens, such as keyhole limpet hemocyanin, bovine serum albumin, tetanus toxoid, etc. *See Microbiology,* Hoeber Medical Division, Harper and Row, 1969; Landsteiner (1962) *Specificity of Serological Reactions,* Dover Publications, New York, and Williams, *et al.* (1967) *Methods in Immunology and Immunochemistry,* Vol. 1, Academic Press, New York for descriptions of methods of preparing polyclonal antisera. A typical method involves hyperimmunization of an animal with an antigen. The blood of the animal is then collected shortly after the repeated immunizations and the gamma globulin is isolated.

In some instances, it is desirable to prepare monoclonal antibodies from various mammalian hosts, such as mice, rodents, primates, humans, etc. Description of techniques for preparing such monoclonal antibodies may be found in, e.g., Stites, *et al*. (eds.) *Basic and Clinical Immunology* (4th ed.), Lange Medical Publications, Los Altos, CA, and references cited therein; Harlow and Lane (1988) *Antibodies: A Laboratory Manual,* CSH Press; Goding (1986) *Monoclonal Antibodies: Principles and Practice* (2d ed.) Academic Press, New York, and particularly in Kohler and Milstein (1975) in *Nature* 256:495-497, which discusses one method of generating monoclonal antibodies.

Summarized briefly, this method involves injecting an animal with an immunogen. The animal is then sacrificed and cells taken from its spleen, which are then fused with myeloma cells. The result is a hybrid cell or "hybridoma" that is capable of reproducing *in vitro*. The population of hybridomas is then screened to isolate individual clones, each of which secrete a single antibody species to the immunogen. In this manner, the individual antibody species obtained are the products of immortalized and cloned single B cells from the immune animal generated in response to a specific site recognized on the immunogenic substance..

Other suitable techniques involve *in vitro* exposure of lymphocytes to the antigenic polypeptides or alternatively to selection of libraries of antibodies in phage or similar vectors. See, Huse, *et al.* (1989) "Generation of a Large Combinatorial Library of the lmmunoglobulin Repertoire in Phage Lambda," *Science 246*:1275-1281; and Ward, *et al*. (1989) *Nature* 341:544-546. The polypeptides and antibodies of the present invention may be used with or without modification, including chimeric or humanized antibodies. Frequently, the polypeptides and antibodies will be labeled by joining, either covalently or non-covalently, a substance which provides for a detectable signal.

A wide variety of labels and conjugation techniques are known and are reported extensively in both the scientific and patent literature. Suitable labels include radionuclides, enzymes, substrates, cofactors, inhibitors, fluorescent moieties, chemiluminescent moieties, magnetic particles, and the like. Patents, teaching the use of such labels include U.S. Patent Nos. 3,817,837; 3,850,752; 3,939,350; 3,996,345; 4,277,437; 4,275,149; and 4,366,241. Also, recombinant immunoglobulins may be produced, see Cabilly, U.S. Patent No. 4,816,567.

The antibodies of this invention can also be used for affinity chromatography in isolating the protein. Columns can be prepared where the antibodies are linked to a solid support, e.g., particles, such as agarose, SEPHA-DEX® , or the like, where a call lysate may be passed through the column, the column washed, followed by increasing concentrations of a mild denaturant, whereby the purified Flt3 ligand protein vall be released..

The antibodies may also be used to screen expression libraries for particular expression products. Usually the antibodies used in such a procedure will be labeled with a moiety allowing easy detection of presence of antigen by antibody binding.

Antibodies raised against each Flt3 ligand will also be useful to raise anti-idiotypic antibodies. These will be useful in detecting or diagnosing various immunological conditions related to expression of the respective antigens.

### Nucleic Acids

The described peptide sequences and the related reagents are useful in isolating a DNA clone encoding Flt3 ligand, e.g., from a natural source. Typically, it will be useful in isolating a gene from mouse, and similar procedures will be applied to isolate genes from other species, e.g., warm blooded animals, such as birds and mammals. See Table 3. Cross hybridization will allow isolation of ligand from other species. A number of different approaches should be available to successfully isolate a suitable nucleic add clone.

The purified protein or defined peptides are useful for generating antibodies by standard methods, as described above. Synthetic peptides or purified protein can be presented to an immune system to generate monoclonal or polyclonal antibodies. See, e.g., Coligan (1991) *Current Protocols in Immunology* Wiley/Greene; and Harlow and Lane (1989) *Antibodies: A Laboratory Manual,* Cold Spring Harbor Press. Alternatively, the Flt3 receptor can be used as a specific binding reagent, and advantage can be taken of its specificity of binding, much like an antibody would be used.

For example, the specific binding composition could be used for screening of an expression library made from a cell line which expresses a Flt3 ligand. The screening can be standard staining of surface expressed ligand, or by panning. Screening of intracellular expression can also be performed by various staining or immunofluorescence procedures. The binding compositions could be used to affinity purify or sort out cells expressing the ligand.

The peptide segments can also be used to predict appropriate oligonucleotides to screen a library. The genetic code can be used to select appropriate oligonucleotides useful as probes for screening. See, e.g., Table 3. In combination with polymerase chain reaction (PCR) techniques, synthetic oligonucleotides will be useful in selecting correct clones from a library. Complementary sequences will also be used as probes or primers. Based upon identification of the likely amino terminus, the third peptide should be particularly useful, e.g., coupled with anchored vector or poly-A complementary PCR techniques or with complementary DNA of other peptides.

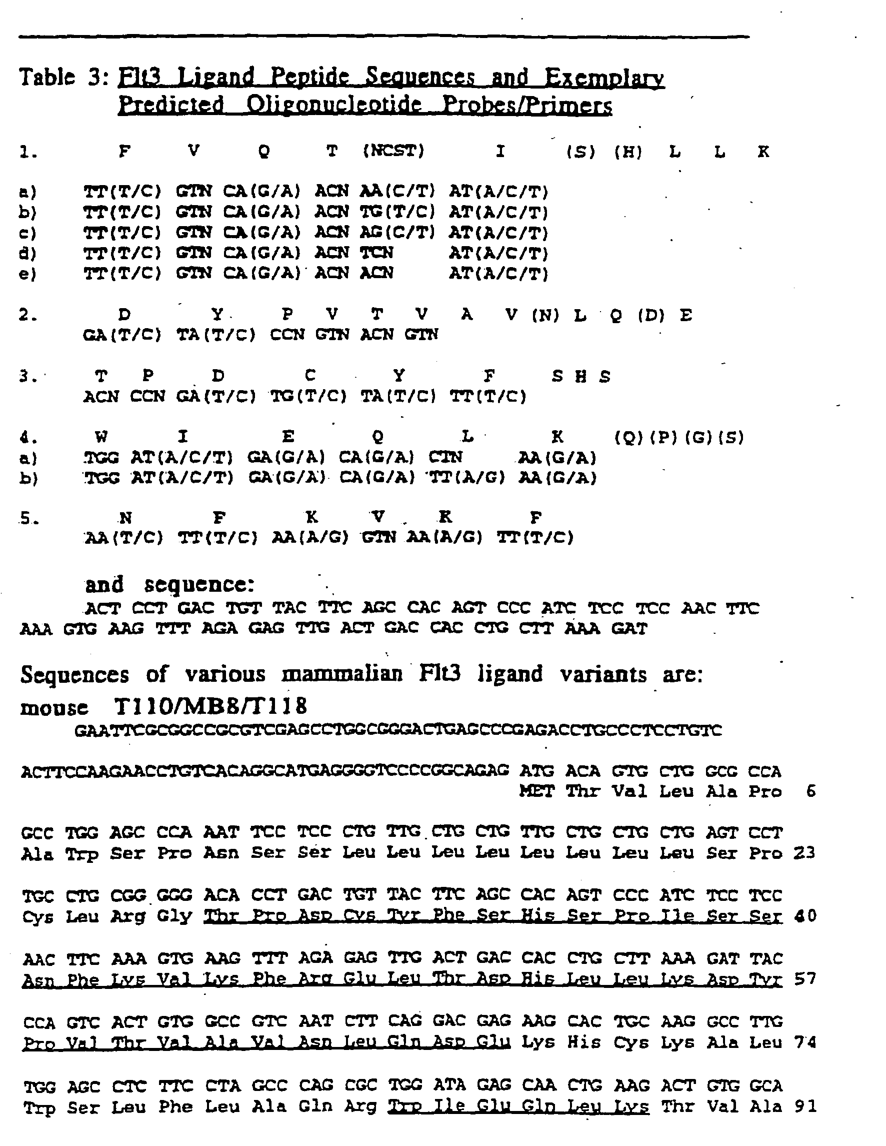

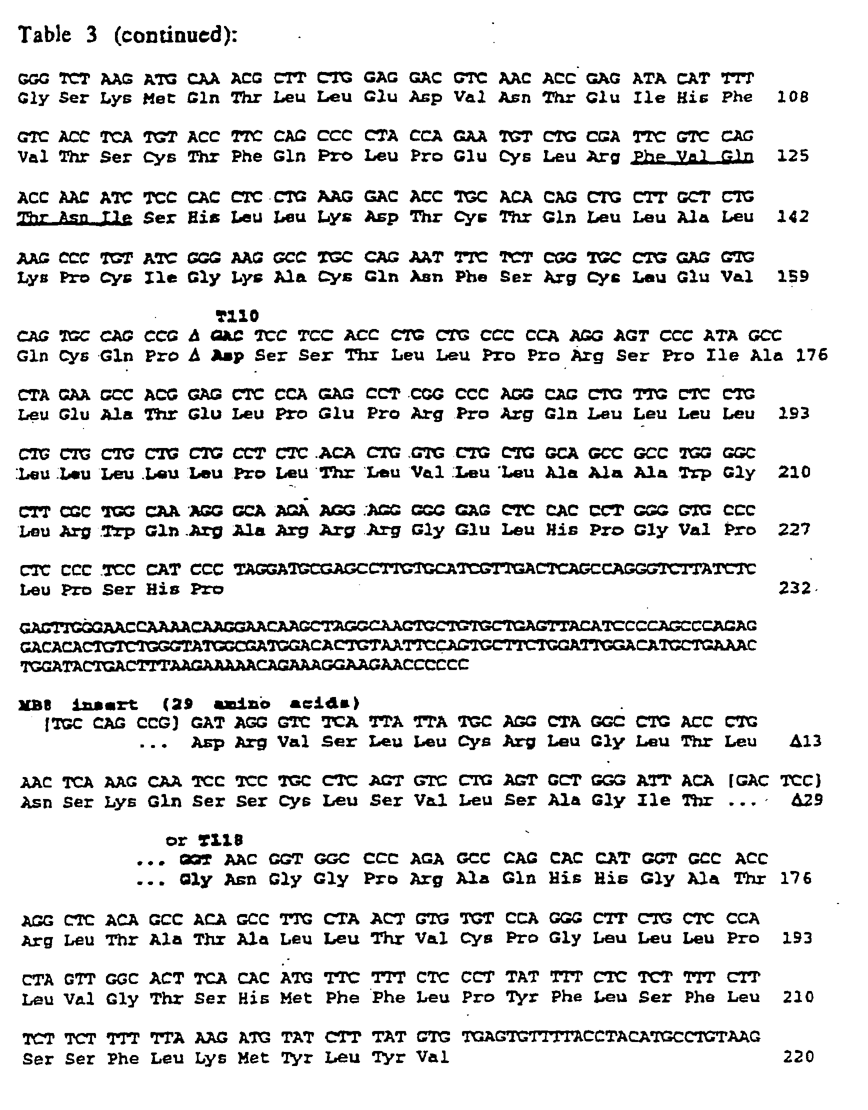

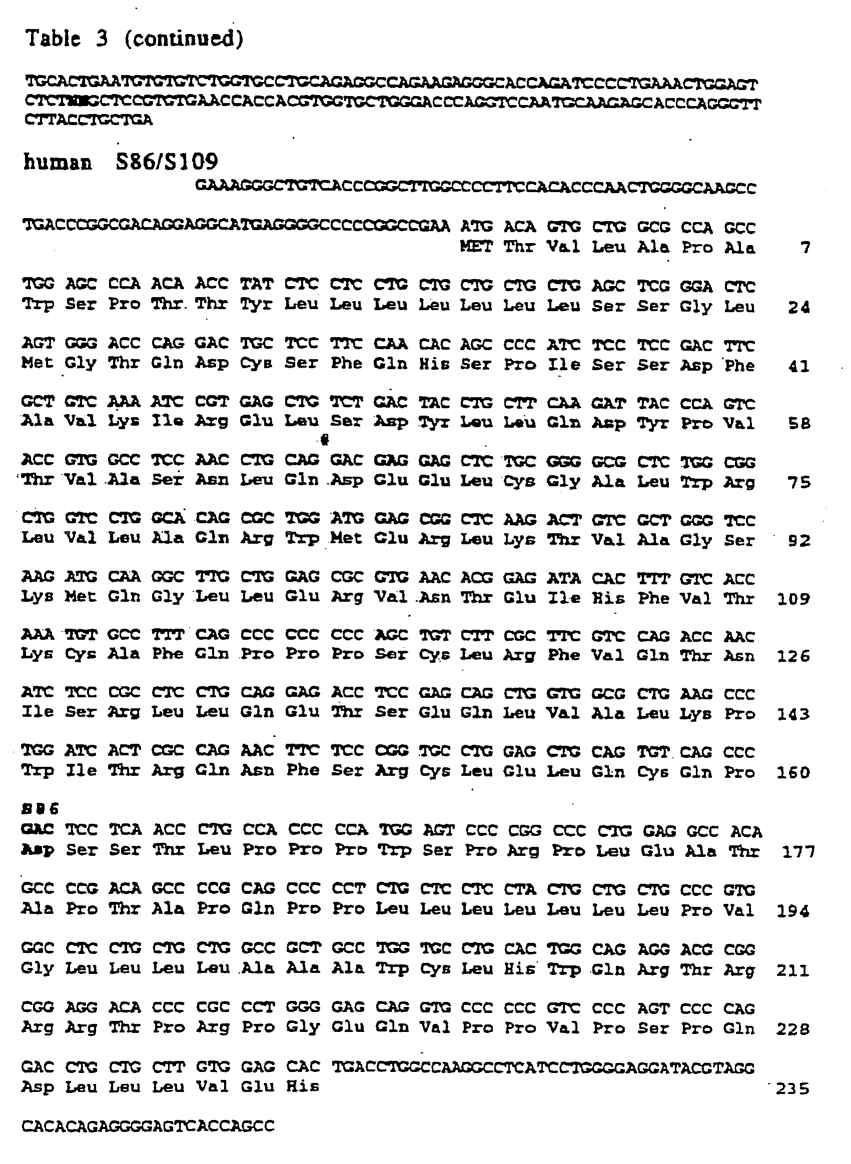

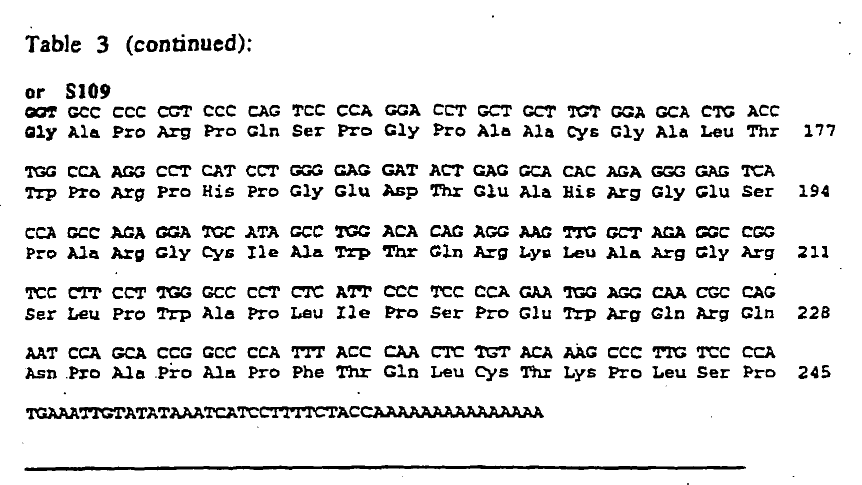

Of course the complementary sequences are also useful.

The sequences shown in Table 3 are also defined in the Sequence Listing as follows:

| Peptide | SEQ ID NO: |
|---|---|
| 1. a) | 25 |
| b) | 26 |
| c) | 27 |
| d) | 28 |
| e) | 29 |
| 2. | 30 |
| 3. | 31 |
| 4. a) | 32 |
| b) | 33 |
| 5. | 34 |
| Nucleotide Sequence | 35 |
| MoT110/T118 | 21 |
| MB8 | 23 |

| T118 | |
|---|---|
| Nucleotide Sequence | 24 |
| Amino Acid Sequence | 22 |
| HuS86/S109 | 19 |
| S109 | 20 |

An isolated nucleic acid encoding an amino terminal segment has been isolated and sequenced and provides the following sequence: ACT CCT GAC TGT TAC TTC AGC CAC AGT CCC ATC TCC TCC AAC TTC AAA GTG AAG TTT AGA GAG TTG ACT GAC CAC CTG CTT AAA GAT. This may be used as a probe to isolate a longer or full length clone and will lead to isolation of other species or allelic variants or other closely related genes. See Table 3 and Table 5, below.

This invention contemplates use of isolated DNA or fragments to encode a biologically active corresponding Flt3 ligand polypeptide. In addition, this invention relates to isolated or recombinant DNA which encodes a biologically active protein or polypeptide which is capable of hybridizing under appropriate conditions with the DNA sequences described herein. Said biologically active protein or polypeptide can be an intact ligand, or fragment, and have an amino acid sequence as disclosed in Table 1. Further, this invention relates to the use of isolated or recombinant DNA, or fragments thereof, which encode proteins which are homologous to a.Ftt3 ligand or which was isolated using cDNA encoding a Flt3 ligand as a probe. The isolated DNA can have the respective regulatory sequences in the 5' and 3' flanks, e.g., promoters, enhancers, poly-A addition signals, and others.

An "isolated" nucleic acid is a nucleic acid, e.g., an RNA, DNA, or a mixed polymer, which is substantially separated from other components which naturally accompany a native sequence, e.g., ribosomes, polymerases, and flanking genomic sequences from the originating species. The term embraces a nucleic acid sequence which has been removed from its naturally occurring environment, and includes recombinant or cloned DNA isolates and chemically synthesized analogs or analogs biologically synthesized by heterologous systems. A substantially pure molecule includes isolated forms of the molecule.

An isolated nucleic acid will generally be a homogeneous composition of molecules, but will, in some embodiments, contain minor heterogeneity. This heterogeneity is typically found at the polymer ends or portions not critical to a desired biological function or activity.

A "recombinant" nucleic acid is defined either by its method of production or its structure. In reference to its method of production, e.g., a product made by a process, the process is use of recombinant nucleic acid techniques, e.g., involving human intervention in the nucleotide sequence, typically selection or production. Alternatively, it can be a nucleic acid made by generating a sequence comprising fusion of two fragments which are not naturally contiguous to each other, but is meant to exclude products of nature, e.g., naturally occurring mutants. Thus, for example, products made by transforming cells with any unnaturally occurring vector is encompassed, as are nucleic acids comprising sequence derived using any synthetic oligonucleotide process. Such is often done to replace a codon with a redundant codon encoding the same or a conservative amino acid, while typically introducing or removing a sequence recognition site.

Alternatively, it is performed to join together nucleic acid segments of desired functions to generate a single genetic entity comprising a desired combination of functions not found in the commonly available natural forms. Restriction enzyme recognition sites are often the target of such artificial manipulations, but other site specific targets, e.g., promoters, DNA replication sites, regulation sequences, control sequences, or other useful features may be incorporated by design. A similar concept is intended for a recombinant, e.g., fusion, polypeptide. Specifically included are synthetic nucleic acids which, by genetic code redundancy, encode polypeptides similar to fragments of these antigens, and fusions of sequences from various different species variants.

A significant "fragment" in a nucleic acid context is a contiguous segment of at least about 17 nucleotides, generally at least 20 nucleotides, more generally at least 23 nucleotides, ordinarily at least 26 nucleofides, more ordinarily at least 29 nucleotides, often at least 32 nucleotides, more often at least 35 nucleotides, typically at least 38 nucleotides, more typically at least 41 nucleotides, usually at least 44 nucleotides, more usually at least 47 nucleofides, preferably at least 50 nucleotides, more preferably at least 53 nucleotides, and in particularly preferred embodiments will be at least 56 or more nucleotides.

A DNA which codes for a Flt3 ligand protein will be particularly useful to identify genes, mRNA, and cDNA species which code for related or homologous ligands, as well as DNAs which code for homologous proteins from different species. There are likely hamologues in other species, including primates. Various Flt3 ligand proteins should be homologous. However, even proteins that have a more distant evolutionary relationship to the ligand can readily be isolated under appropriate conditions using these sequences if they are sufficiently homologous. Primate Flt3 ligand proteins are of particular interest

This invention further relates to recombinant DNA molecules and fragments having a DNA sequence identical to or highly homologous to the isolated DNAs set forth herein. In particular, the sequences will often be operably linked to DNA segments which control transcription, translation, and DNA replication. Alternatively, recombinant clones derived from the genomic sequences, e.g., containing introns, will be useful for transgenic studies, including, e.g., transgenic cells and organisms, and for gene therapy. See, e.g., Goodnow (1992) "Transgenic Animals" in Roitt (ed.) *Encyclopedia of Immunology* Academic Press, San Diego, pp. 1502-1504; Travis (1992) *Science* 256:1392-1394; Kuhn, *et al.* (1991) *Science* 254:707-710; Capecchi (1989) *Science* 244:1288; Robertson (1987)(ed.) *Teratocarcinomas and Embryonic Stem Cells: A Practical Approach* IRL Press, Oxford; and Rosenberg (1992) *J. Clinical Oncology 10*:180-199.

Homologous nucleic acid sequences, when compared, exhibit significant similarity. The standards for homology in nucleic acids are either measures for homology generally used in the art by sequence comparison or based.upon hybridization conditions. The hybridization conditions are described in greater detail below.

Substantial homology in the nucleic acid sequence comparison context means either that the segments, or their complementary strands, when compared, are identical when optimally aligned, with appropriate nucleotide insertions or deletions, in at least about 50% of the nucleotides, generally at least 56%, more generally at least 59%, ordinarily at least 62%, more ordinarily at least 65%, often at least 68%, more often at least 71%, typically at least 74%, more typically at least 77%, usually at least 80%, more usually at least about 85%, preferably at least about 90%, more preferably at least about 95 to 98% or more, and in particular embodiments, as high at about 99% or more of the nucleotides.

Alternatively, substantial homology exists when the segments will hybridize under selective hybridization conditions, to a strand, or its complement, typically using a sequence derived from Table 2. Typically, selective hybridization will occur when there is at least about 55% homology over a stretch of at least about 30 nucleotides, preferably at least about 65% over a stretch of at least about 25 nucleotides, more preferably at least about 75%, and most preferably at least about 90% over about 20 nucleotides. See, Kanehisa (1984) *Nuc. Acids Res. 12*:203-213.

The length of homology comparison, as described, may be over longer stretches, and in certain embodiments will be over a stretch of at least about 17 nucleotides, usually at least about 20 nucleotides, more usually at least about 24 nucleotides, typically at least about 28 nucleotides, more typically at least about 40 nucleotides, preferably at least about 50 nucleotides, and more preferably at least about 75 to 100 or more nucleotides.

Stringent conditions, in referring to homology in the hybridization context, will be stringent combined conditions of salt, temperature, organic solvents, and other parameters, typically those controlled in hybridization reactions. Stringent temperature conditions will usually include temperatures in excess of about 30° C, more usually in excess of about 37° C, typically in excess of about 45° C, more typically in excess of about 55° C, preferably in excess of about 65° C, and more preferably in excess of about 70° C. Stringent salt conditions will ordinarily be less than about 1000 mM, usually less than about 500 mM, more usually less than about 400 mM, typically less than about 300 mM, preferably less than about 200 mM, and more preferably less than about 150 mM. However, the combination of parameters is much more important than the measure of any single parameter. See, e.g., Wetmur and Davidson (1968) *J. Mol. Biol. 31*:349-370.

Flt3 ligand from other mammalian species can be cloned and isolated by cross-species hybridization of closely related species. See, e.g., below. Homology may be relatively low between distantly related species, and thus hybridization of relatively closely related species is advisable. Alternatively, preparation of an antibody preparation which exhibits less species specificity may be useful in expression cloning approaches.

### Making Flt3 Ligand: Mimetics

DNA which encodes the Flt3 ligand or fragments thereof can be obtained by chemical synthesis, screening cDNA libraries, or by screening genomic libraries prepared from a wide variety of cell lines or tissue samples.

This DNA can be expressed in a wide variety of host cells for the synthesis of a full-length ligand or fragments which can in turn, for example, be used to generate polyclonal or monoclonal antibodies; for binding studies; for construction and expression of modified molecules; and for structure/function studies. Each antigen or its fragments can be expressed in host cells that are transformed or transfected with appropriate expression vectors. These molecules can be substantially purified to be free of protein or cellular contaminants, other than those derived from the recombinant host, and therefore are particularly useful in pharmaceutical compositions when combined with a pharmaceutically acceptable carrier and/or diluent The antigen, or portions thereof, may be expressed as fusions with other proteins.

Expression vectors are typically self-replicating DNA or RNA constructs containing the desired antigen gene or its fragments, usually operably linked to suitable genetic control elements that are recognized in a suitable host cell. These control elements are capable of effecting expression within a suitable host. The specific type of control elements necessary to effect expression will depend upon the eventual host cell used. Generally, the genetic control elements can include a prokaryotic promoter system or a eukaryotic promoter expression control system, and typically include a transcriptional promoter, an optional operator to control the onset of transcription, transcription enhancers to elevate the level of mRNA expression, a sequence that encodes a suitable ribosome binding site, and sequences that terminate transcription and translation. Expression vectors also usually contain an origin of replication that allows the vector to replicate independently of the host cell.

The vectors of this invention contain DNA which encodes a Flt3 ligand of the second aspect, typically encoding a biologically active polypeptide. The DNA can be under the control of a viral promoter and can encode a selection marker. This invention further contemplates use of such expression vectors which are capable of expressing eukaryotic cDNA coding for a Ftt3 ligand in a prokaryotic or eukaryotic host, where the vector is compatible with the-host and where the eukaryotic cDNA coding for the ligand is inserted into the vector such that growth of the host containing the vector expresses the cDNA in question. Usually, expression vectors are designed for stable replication in their host cells or for amplification to greatly increase the total number of copies of the desirable gene per cell. It is not always necessary to require that an expression vector replicate in a host cell, e.g., it is possible to effect transient expression of the ligand or its fragments in various hosts using vectors that do not contain a replication origin that is recognized by the host cell. It is also possible to use vectors that cause integration of a Ftt3 ligand gene or its fragments into the host DNA by recombination, or to integrate a promoter which controls expression of an endogenous gene.

Vectors, as used herein, comprise plasmids, viruses, bacteriophage, integratable DNA fragments, and other vehicles which enable the integration of DNA fragments into the genome of the host. Expression vectors are specialized vectors which contain genetic control elements that effect expression of operably linked genes. Plasmids are the most commonly used form of vector but all other forms of vectors which serve an equivalent function and which are, or become, known in the art are suitable for use herein. See, e.g., Pouwels, *et al.* (1985 and Supplements) *Cloning Vectors:* A *Laboratory Manual,* Elsevier, N.Y, and Rodriquez, *et al.* (1988)(eds.) *Vectors: A Survey of Molecular Cloning Vectors and Their Uses,* Buttersworth, Boston, MA.

Transformed cells include cells, preferably mammalian, that have been transformed or transfected with Flt3 ligand gene containing vectors constructed using recombinant DNA techniques. Transformed host cells usually express the ligand or its fragments, but for purposes of cloning, amplifying, and manipulating its DNA, do not need to express the protein. This invention further contemplates culturing transformed cells in a nutrient medium, thus permitting the protein to accumulate in the culture. The protein can be recovered, either from the culture or from the culture medium

For purposes of this invention, DNA sequences are operably linked when they are functionally related to each other. For example, DNA for a presequence or secretory leader is operably linked to a polypeptide if it is expressed as a preprotein or participates in directing the polypeptide to the cell membrane or in secretion of the polypeptide. A promoter is operably linked to a coding sequence if it controls the transcription of the polypeptide; a ribosome binding site is operably linked to a coding sequence if it is positioned to permit translation. Usually, operably linked means contiguous and in reading frame, however, certain genetic elements such as repressor genes are not contiguously linked but still bind to operator sequences that in turn control expression.

Suitable host cells include prokaryotes, lower eukaryotes, and higher eukaryotes. Prokaryotes include both gram negative and gram positive organisms, e.g., *E. coli* and *B. subtilis.* Lower eukaryotes include yeasts, e.g., *S. cerevisiae* and *Pichia,* and species of the genus *Dictyostelium.* Higher eukaryotes include established tissue culture cell lines from animal cells, both of non-mammalian origin, e.g., insect cells, and birds, and of mammalian origin, e.g., human, primates, and rodents.

Prokaryotic host-vector systems include a wide variety of vectors for many different species. As used herein, *E. coli* and its vectors will be used generically to include equivalent vectors used in other prokaryotes. A representative vector for amplifying DNA is pBR322 or many of its derivatives. Vectors that can be used to express the Flt3 ligands or its fragments include, but are not limited to, such vectors as those containing the lac promoter (pUC-series); trp promoter (pBR322-trp); lpp promoter (the pIN-series); lambda-pP or pR promoters (pOTS); or hybrid promoters such as ptac (pDR540). See Brosius, *et al.* (198B) "Expression Vectors Employing Lambda-, trp-, lac-, and Ipp-derived Promoters", in Rodriguez and Denhardt (eds.) *Vectors: A Survey of Molecular Cloning Vectors and Their Uses.* Buttersworth, Boston, Chapter 10, pp. 205-236.

Lower eukaryotes, e.g., yeasts and Dictyostelium, may be transformed with Fit3 ligand sequence containing vectors. For purposes of this invention, the most common lower eukaryotic host is the bakers yeast, Saccharomyces cerevisiae. It will be used to generically represent lower eukaryotes although a number of other strains and species are also available. Yeast vectors typically consist of a replication origin (unless of the integrating type), a selection gene, a promoter, DNA encoding the desired protein or its fragments, and sequences for translation termination, polyadenyla tion, and transcription termination.

Suitable expression vectors for yeast include such constitutive promoters as 3-phosphoglycerate kinase and various other glycolytic enzyme gene promoters or such inducible promoters as the alcohol dehydrogenase 2 promoter or metallothionine promoter. Suitable vectors include derivatives of the following types: self-replicating low copy number (such as the YRp-series), self-repficating high copy number (such as the YEp-series): integrating types (such as the Ylp-series), or mini-chromosomes (such as the YCp-series).

Higher eukaryotic tissue culture cells are the preferred host cells for expression of the functionally active Flt3 ligand protein. In principle, any higher eukaryotic tissue culture cell line is workable, e.g., insect baculovirus expression systems, whether from an invertebrate or vertebrate source. However, mammalian cells are preferred, in that the processing, both cotranslationally and posttranslationally. Transformation or transfection and propagation of such cells has become a routine procedure.

Examples of useful cell lines include HeLa cells, Chinese hamster ovary (CHO) cell lines, baby rat kidney (BRK) cell lines, insect cell lines, bird cell lines, and monkey (COS) cell lines. Expression vectors for such cell lines usually include an origin of replication, a promoter, a translation initiation site, RNA splice sites (if genomic DNA is used), a polyadenylation site, and a transcription termination site. These vectors also usually contain a selection gene or amplification gene.

Suitable expression vectors may be plasmids, viruses, or retroviruses carrying promoters derived, e.g., from such sources as from adenovirus, SV40, parvoviruses, vaccinia virus, or cytomegalovirus. Representative examples of suitable expression vectors include pCDNA1; pCD, see Okayama, *et al*. (1985) *Mol. Cell Biol. 5*:1136-1142; pMC1 neo Poly-A, see Thomas, *et al.* (1987) *Cell 51*:503-512; and a baculovirus vector such as pAC 373 or pAC 610.

It will often be desired to express a FIt3 ligand polypeptide in a system which provides a specific or defined glycosylation pattern. In this case, the usual pattern will be that provided naturally by the expression system. However, the pattern will be modifiable by exposing the polypeptide, e.g., an unglycosylated form, to appropriate glycosylating proteins introduced into a heterologous expression system. For example, the Flt3 ligand gene may be co-transformed with one or more genes encoding mammalian or other glycasylating enzymes. Using this approach, certain mammalian glycosylation patterns will be achievable or approximated in prokaryote or other cells.

The Flt3 ligand, or a fragment thereof, may be engineered to be phosphatidyl inositol (PI) linked to a cell membrane, but can be removed from membranes by treatment with a phosphatidyl inositol cleaving enzyme, e.g., phosphatidyl inositol phospholipase-C. This releases the antigen in a biologically active form, and allows purification by standard procedures of protein chemistry. See, e.g., Low (1989) *Biochim. Biophys. Acta* 988:427-454; Tse, *et al.* (1985) *Science* 230:1003-1008; and Brunner, *et al*. (1991) *J. Cell Biol. 114*:1275-1283.

Now that the Flt3 ligand has been characterized, fragments or derivatives thereof can be prepared by conventional processes for synthesizing peptides. These include processes such as are described in Stewart and Young (1984) *Solid Phase Peptide Synthesis,* Pierce Chemical Co., Rockford, IL; Bodanszky and Bodanszky (1984) *The Practice of Peptide Synthesis,* Springer-Verlag, New York, and Bodanszky (1984) *The Principles of Peptide* Synthesis, Spcinger-Verlag, New York.

For example, an azide process, an acid chloride process, an acid anhydride process, a mixed anhydride process, an active ester process (for example, p-nitrophenyl ester, N-hydroxysuccinimide ester, or cyanomethyl ester), a carbodiimidazole process, an oxidative-reductive process, or a dicyclohexylcarbodiimide (DCCD)/additive process can be used. Solid phase and solution phase syntheses are both applicable to the foregoing processes.

The Flt3 ligand, fragments, or derivatives are suitably prepared in accordance with the above processes as typically employed in peptide synthesis, generally either by a so-called stepwise process which comprises condensing an amino acid to the terminal amino acid, one by one in sequence, or by coupling peptide fragments to the terminal amino add. Amino groups that are not being used in the coupling reaction are typically protected to prevent coupling at an incorrect location.

If a solid phase synthesis is adopted, the C-terminal amino acid is bound to an insoluble carrier or support through its carboxyl group. The insoluble carrier is not particularly limited as long as it has a binding capability to a reactive carboxyl group. Examples of such insoluble carriers include halomethyl resins, such as chloromethyl resin or bromomethyl resin, hydroxymethyl resins, phenol resins, tert-alkyloxycarbonyl-hydrazidated resins, and the like.

An amino group-protected amino acid is bound in sequence through condensation of its activated carboxyl group and the reactive amino group of the previously formed peptide or chain, to synthesize the peptide step by step. After synthesizing the complete sequence, the peptide is split off from the insoluble carrier to produce the peptide. This solid-phase approach is generally described by Merrifield, *et al.* (1963) in *J. Am. Chem. Soc. 85:*2149-2156.

The prepared ligand and fragments thereof can be isolated and purified from the reaction mixture by means of peptide separation, for example, by extraction, precipitation, electrophoresis and various forms of chromatography, and the like. The Flt3 ligands of this invention can be obtained in varying degrees of purity depending upon its desired use. Purification can be accomplished by use of the protein purification techniques disclosed herein or by the use of the antibodies herein described in immunoabsorbant affinity chromatography.

This immunoabsorbant affinity chromatography is carried out by first linking the antibodies to a solid support and then contacting the linked antibodies with solubilized lysates of appropriate source cells, lysates of other cells expressing the ligand, or lysates or supernatants of cells producing the Flt3 ligand as a result of DNA techniques, see below.

### Uses

The present invention provides reagents which will find use in diagnostic applications as described elsewhere herein, e.g., in the general description for developmental abnormalities, or below in the description of kits for diagnosis.

This invention also provides reagents with significant therapeutic value. The Flt3 ligand (naturally occurring or recombinant), fragments thereof and antibodies thereto, along with compounds identified as having binding affinity to Ftt3 ligand, should be useful in the treatment of conditions associated with abnormal physiology or development, including abnormal proliferation, e.g., cancerous conditions, or degenerative conditions. In particular, modulation of development of lymphoid cells is likely, but the wider tissue distribution on non-lymphoid tissues, e.g., gonads and neural cells, suggests that development of those tissues will be similarly responsive.

Abnormal proliferation, regeneration, degeneration, and atrophy may be modulated by appropriate therapeutic treatment using the compositions provided herein. For example, a disease or disorder associated with abnormal expression or abnormal signaling by a Flt3 ligand should be a likely target for an agonist or antagonist of the ligand. The ligand likely plays a role in regulation or development of hematopoietic cells, e.g., lymphoid cells, which affect immunological responses, e.g., autoimmune disorders.

Other abnormal developmental conditions are known in each of the cell types shown to possess Flt3 receptor mRNA by Northern blot analysis. See Berkow (ed.) *The Merck Manual of Diagnosis and Therapy,* Merck & Co., Rahway. N.J.; and Thorn, *et al. Harrison's Principles of Internal Medicine.* McGraw-Hill, N.Y. For example, neural and brain abnormalities exist in, e.g., cerebrovascular disease, CNS neoplasms, demyelinating diseases, and muscular dystrophies. Liver disorders, kidney disorders, cardiopulmonary disorders, and other problems often cause medical symptoms. These problems may be susceptible to prevention or treatment using compositions provided herein.

Recombinant Flt3 or Flt3 ligand antibodies can be purified and then administered to a patient These reagents can be combined for therapeutic use with additional active or inert ingredients, e.g., in conventional pharmaceutically acceptable carriers or diluents, e.g., immunogenic adjuvants, along with physiologically innocuous stabilizers and excipients. These combinations can be sterile filtered and placed into dosage forms as by lyophilization in dosage vials or storage in stabilized aqueous preparations. This invention also contemplates use of antibodies or binding fragments thereof, including forms which are not complement binding.

Drug screening using Flt3 receptor or fragments thereof can be performed to identify compounds having binding affinity to Flt3 ligand, including isolation of associated components. Subsequent biological assays can then be utilized to determine if the compound has intrinsic stimulating activity and is therefore a blacker or antagonist in that it blocks the activity of the ligand. Likewise, a compound having intrinsic stimulating activity can activate the receptor and is thus an agonist in that it simulates the activity of Flt3 ligand. This invention further contemplates the therapeutic use of antibodies to Flt3 ligand as antagonists. This approach should be particularly useful with other Flt3 ligand species variants.

The quantities of reagents necessary for effective therapy will depend upon many different factors, including means of administration, target site, physiological state of the patient, and other medicaments administered. Thus, treatment dosages should be titrated to optimize safety and efficacy. Typically, dosages used *in vitro* may provide useful guidance in the amounts useful for *in situ* administration of these reagents. Animal testing of effective doses for treatment of particular disorders will provide further predictive indication of human dosage.

Various considerations are described, e.g., in Gilman, *et al.* (eds.) (1990) *The Pharmacological Bases of Therapeutics,* 8th Ed., Pergamon Press; and *Remington's Pharmaceutical Sciences,* 17th ed. (1990), Mack Publishing Co., Easton, Penn. Methods for administration are discussed therein and below, e.g., for oral, intravenous, intraperitoneal, or intramuscular administration, transdermal diffusion, and others.

Pharmaceutically acceptable carriers will include water, saline, buffers, and other compounds described, e.g., in the *Merck Index,* Merck & Co., Rahway, New Jersey. Dosage ranges would ordinarily be expected to be in amounts lower than 1 mM concentrations, typically less than about 10 µM concentrations, usually less than about 100 nM, preferably less than about 10 pM (picomalar), and most preferably less than about 1 fM (femtomolar), with an appropriate carrier. Slow release formulations, or a slow release apparatus will often be utilized for continuous administration.

Flt3 ligand, fragments thereof, and antibodies to it or its fragments, antagonists, and agonists, may be administered directly to the host to be treated or, depending on the size of the compounds, it may be desirable to conjugate them to carrier proteins such as ovalbumin or serum albumin prior to their administration. Therapeutic formulations may be administered in any conventional dosage formulation.

White it is possible for the active ingredient to be administered alone, it is preferable to present it as a pharmaceutical formulation. Formulations typically comprise at least one active ingredient, as defined above, together with one or more acceptable carriers thereof. Each carrier should be both pharmaceutically and physiologically acceptable in the sense of being compatible with the other ingredients and not injurious to the patient Formulations include those suitable for oral, rectal, nasal, or parenteral (including subcutaneous, intramuscular, intravenous and intradermal) administration.

The formulations may conveniently be presented in unit dosage form and may be prepared by any methods well known in the art of pharmacy. See, e.g., Gilman, *et al.* (eds.) (1990) *The Pharmacological Bases of Therapeutics, supra;* and *Remington's Pharmaceutical Sciences, supra;* Avis, *et al.* (eds.) (1993) *Pharmaceutical Dosage Forms: Parenteral Medications* Dekker, New York; Lieberman, *et al.* (eds.) (1990) *Pharmaceutical Dosgae Forms: Tablets,* Dekker, New York; and Lieberman, *et al.* (eds.) (1990) *Pharmaceutical Dosage Forms: Disperse Systems,* Dekker, New York The therapy of this invention may be combined with or used in association with other chemotherapeutic or chemopreventive agents.

Both the naturally occurring and the recombinant form of the Flt3 ligands of this invention are particularly useful in kits and assay methods which are capable of screening compounds for binding activity to the proteins. Several methods of automating assays have been developed in recent years so as to permit screening of tens of thousands of compounds in a short period. See, e.g., Fodor, *et al.* (1991) *Science 251*:767-773 which describes means for testing of binding affinity by a plurality of defined polymers synthesized on a solid substrate. The development of suitable assays can be greatly facilitated by the availability of large amounts of purified, soluble Flt3 ligand as provided by this invention.

For example, antagonists can normally be found once the ligand has been structurally defined. Testing of potential ligand analogs is now possible upon the development of highly automated assay methods using a purified Ftt3 receptor. In particular, new agonists and antagonists will be discovered by using screening techniques described herein. Of particular importance are compounds found to have a combined binding affinity for multiple Flt3 receptors. e.g., compounds which can serve as antagonists for species variants of Ftt3 ligand.

This invention is particularly useful for screening compounds by using recombinant receptor in any of a variety of drug screening techniques. The advantages of using a recombinant protein in screening for specific ligands include: (a) improved renewable source of the Ftt3 receptor from a specific source; (b) potentially greater number of ligands per cell giving better signal to noise ratio in assays; and (c) species variant specificity (theoretically giving greater biological and disease specificity).

One method of drug screening utilizes eukaryotic or prokaryotic host cells which are stably transformed with recombinant DNA molecules expressing the Flt3 receptor. Cells may be isolated which express a receptor in isolation from any others. Such cells, either in viable or fixed form, can be used for standard ligand/receptor binding assays. See also, Parce, *et al.* (1989) *Science 246*:243-247; and Owicki, *et al*. (1990) *Proc. Nat'l Acad Sci.* USA 87:4007-4011 describe sensitive methods to detect cellular responses.

Competitive assays are particularly useful, where the cells (source of Flt3 ligand) are contacted and incubated with a labeled receptor or antibody having known binding affinity to the ligand, such as ¹²⁵I-antibody, and a test sample whose binding affinity to the binding composition is being measured. The bound and free labeled binding compositions are then separated to assess the degree of ligand binding.

The amount of test compound bound is inversely proportional to the amount of labeled receptor binding to the known source. Any one of numerous techniques can be used to separate bound from free ligand to assess the degree of ligand binding. This separation step could typically involve a procedure such as adhesion to filters followed by washing, adhesion to plastic followed by washing, or centrifugation of the cell membranes. Viable cells could also be used to screen for the effects of drugs on Fft3 ligand mediated functions, e.g., second messenger levels, i.e., Ca⁺⁺; cell proliferation; inositol phosphate pool changes; and others. Some detection methods allow for elimination of a separation step, e.g., a proximity sensitive detection system. Calcium sensitive dyes will be useful for detecting Ca⁺⁺ levels, with a fluorimeter or a fluorescence cell sorting apparatus.

Another method utilizes membranes from transformed eukaryotic or prokaryotic host cells as the source of the Flt3 ligand These cells are stably transformed with DNA vectors directing the expression of a Flt3 ligand, e.g., an engineered membrane bound form. Essentially, the membranes would be prepared from the cells and used in any receptor/ligand binding assay such as the competitive assay set forth above.

Still another approach is to use solubilized, unpurified or solubilized, purified Flt3 ligand from transformed eukaryotic or prokaryotic host cells. This allows for a "molecular" binding assay with the advantages of increased specificity, the ability to automate, and high drug test throughput

Another technique for drug screening involves an approach which provides high throughput screening for compounds having suitable binding affinity to Flt3 receptor and is described in detail in Geysen, European Patent Application 84/03564, published on September 13, 1984. First, large numbers of different small peptide test compounds are synthesized on a solid substrate, e.g., plastic pins or some other appropriate surface. Then all the pins are reacted with solubilized, unpurified or solubilized, purified Flt3 receptor, and washed. The next step involves detecting bound Flt3 receptor.

Rational drug design may also be based upon structural studies of the molecular shapes of the Flt3 ligand and other effectors or analogs. Effectors may be other proteins which mediate other functions in response to ligand binding, or other proteins which normally interact with the receptor. One means for determining which sites interact with specific other proteins is a physical structure determination, e.g., x-ray crystallography or 2 dimensional NMR techniques. These will provide guidance as to which amino acid residues form molecular contact regions. For a detailed description of protein structural determination, see, e.g., Blundell and Johnson (1976) *Protein Crystallography,* Academic Press, New York

Purified Flt3 ligand can be coated directly onto plates for use in the aforementioned drug screening techniques. However, non-neutralizing antibodies to these ligands can be used as capture antibodies to immobilize the respective ligand on the solid phase.

### Kits

This invention also contemplates use of Flt3 ligand proteins, fragments thereof, peptides, and their fusion products in a variety of diagnostic kits and methods for detecting the presence of ligand or a Flt3 receptor. Typically the kit will have a compartment containing either a defined Flt3 ligand peptide or gene segment or a reagent which recognizes one or the other, e.g., receptor fragments or antibodies.

A kit for determining the binding affinity of a test compound to a Flt3 ligand would typically comprise a test compound; a labeled compound, for example a receptor or antibody having known binding affinity for the ligand; a source of Flt3 ligand (naturally occurring or recombinant); and a means for separating bound from free labeled compound, such as a solid phase for immobilizing the ligand. Once compounds are screened, those having suitable binding affinity to the ligand can be evaluated in suitable biological assays, as are well known in the art, to determine whether they act as agonists or antagonists to the receptor. The availability of recombinant Flt3 ligand polypeptides also provide well defined standards for calibrating such assays.

A preferred kit for determining the concentration of, for example, a Flt3 ligand in a sample would typically comprise a labeled compound, e.g., receptor or antibody, having known binding affinity for the ligand, a source of ligand (naturally occurring or recombinant) and a means for separating the bound from free labeled compound, for example, a solid phase for immobilizing the Flt3 ligand. Compartments containing reagents, and instructions, will normally be provided.

Antibodies, including antigen binding fragments, specific for the Flt3 ligand or ligand fragments are useful in diagnostic applications to detect the presence of elevated levels of Flt3 ligand and/or its fragments. Such diagnostic assays can employ lysates, live cells, fixed cells, immunofluorescence, cell cultures, bodyfluids, and further can involve the detection of antigens related to the ligand in serum, or the like. Diagnostic assays may be homogeneous (without a separation step between free reagent and antigen-figand complex) or heterogeneous (with a separation step). Various commercial assays exist, such as radioimmunoassay (RIA), enzyme-linked immunosorbent assay (ELISA), enzyme immunoassay (EIA), enzyme-multiplied immunoassay technique (EMIT), substrate-labeled fluorescent immunoassay (SLFIA), and the like. For example, unlabeled antibodies can be employed by using a second antibody which is labeled and which recognizes the antibody to a Flt3 ligand or to a particular fragment thereof. Similar assays have also been extensively discussed in the literature. See, e.g., Harlow and Lane (1988) *Antibodies: A Laboratory Manual,* CSH.

Anti-idiotypic antibodies may have similar use to diagnose presence of antibodies against a Flt3 ligand, as such may be diagnostic of various abnormal states. For example, overproduction of Flt3 ligand may result in production of various immunological reactions which may be diagnostic of abnormal physiological states, particularly in proliferative cell conditions such as cancer or abnormal differentiation.

Frequently, the reagents for diagnostic assays are suppfied in kits, so as to optimize the sensitivity of the assay. For the subject invention, depending upon the nature of the assay, the protocol, and the label, either labeled or unlabeled antibody or receptor, or labeled Flt3 ligand is provided. This is usually in conjunction with other additives, such as buffers, stabilizers, materials necessary for signal production such as substrates for enzymes, and the like. Preferably, the kit will also contain instructions for proper use and disposal of the contents after use. Typically the kit has compartments for each useful reagent Desirably, the reagents are provided as a dry lyophilized powder, where the reagents may be reconstituted in an aqueous medium providing appropriate concentrations of reagents for performing the assay.

Any of the aforementioned constituents of the drug screening and the diagnostic assays may be used without modification or may be modified in a variety of ways. For example, labeling may be achieved by covalently or non-covalently joining a moiety which directly or indirectly provides a detectable signal. In any of these assays, the ligand, test compound, Flt3 ligand, or antibodies thereto can be labeled either directly or indirectly. Possibilities for direct labeling include label groups: radiolabels such as ¹²⁵I, enzymes (U.S. Pat No. 3,645,090) such as peroxidase and alkaline phosphatase, and fluorescent labels (U.S. Pat. No. 3,940,475) capable of monitoring the change in fluorescence intensity, wavelength shift, or fluorescence polarization. Possibilities for indirect labeling include biotinylation of one constituent followed by binding to avidin coupled to one of the above label groups.

There are also numerous methods of separating the bound from the free ligand, or alternatively the bound from the free test compound. The Flt3 ligand can be immobilized on various matrixes followed by washing. Suitable matrixes include plastic such as an ELISA plate, filters, and beads. Methods of immobilizing the Flt3 ligand to a matrix include, without limitation, direct adhesion to plastic, use of a capture antibody, chemical coupling, and biotin-avidin. The last step in this approach involves the precipitation of ligand/receptor or ligand/antibody complex by any of several methods including those utilizing, e.g., an organic solvent such as polyethylene glycol or a salt such as ammonium sultate. Other suitable separation techniques include, without limitation, the fluorescein antibody magnetizable particle method described in Rattle, *et al.* (1984) *Clin. Chem. 30*:1457-1461, and the double antibody magnetic particle separation as described in U.S. Pat No. 4,659,678.

Methods for linking proteins or their fragments to the various labels have been extensively reported in the literature and do not require detailed discussion here. Many of the techniques involve the use of activated carboxyl groups either through the use of carbodiimide or active esters to form peptide bonds, the formation of thioethers by reaction of a mercapto group with an activated halogen such as chloroacetyl, or an activated olefin such as maleimide, for linkage, or the like. Fusion proteins will also find use in these applications.

Another diagnostic aspect of this invention involves use of oligonucleotide or polynucleotide sequences taken from the sequence of a Flt3 ligand. These sequences can be used as probes for detecting levels of the ligand message in samples from patients suspected of having an abnormal condition, e.g., cancer or developmental problem-The preparation of both RNA and DNA nucleotide sequences, the labeling of the sequences, and the preferred size of the sequences has received ample description and discussion in the literature. Normally an oligonucleotide probe should have at least about 14 nucleotides, usually at least about 18 nucleotides, and the polynucleotide probes may be up to several kilobases. Various labels may be employed, most commonly radionuclides, particularly ³²P.

However, other techniques may also be employed, such as using biotin modified nucleotides for introduction into a polynucleotide. The biotin then serves as the site for binding to avidin or antibodies, which may be labeled with a wide variety of labels, such as radionuclides, fluorescers, enzymes, or the like. Alternatively, antibodies may be employed which can recognize specific duplexes, including DNA duplexes, RNA duplexes. DNA-RNA hybrid duplexes, or DNA-protein duplexes. The antibodies in turn may be labeled and the assay carried out where the duplex is bound to a surface, so that upon the formation of duplex on the surface, the presence of antibody bound to the duplex can be detected. The use of probes to the novel anti-sense RNA may be carried out in any conventional techniques such as nucleic acid hybridization, plus and minus screening, recombinational probing, hybrid released translation (HRT), and hybrid arrested translation (HART). This also includes amplification techniques such as polymerase chain reaction (PCR).

Diagnostic kits which also test for the qualitative or quantitative presence of other markers are also contemplated. Diagnosis or prognosis may depend on the combination of multiple indications used as markers. Thus, kits may test for combinations of markers. See, e.g., Viallet, *et al.* (1989) *Progress in Growth Factor Res.* 1:89-97.

### EXAMPLES

The broad scope of this invention is best understood with reference to the following examples, which are not intended to illustrate but not to limit the invention to specific embodiments.

### General Methods

Some of the standard methods are described or referenced. e.g., in Maniatis, *et al.* (1982) *Molecular Cloning,* A *Laboratory Manual,* Cold Spring Harbor Laboratory, Cold Spring Harbor Press; Sambrook. *et al.* (1989) *Molecular Cloning: A.Laboratory Manual,* (2d ed.), vols 1-3, CSH Press, NY; Ausubel, *et al., Biology,* Greene Publishing Associates. Brooklyn, NY; or Ausubel, *et al.* (1987 and Supplements) *Current Protocols in Molecular Biology*, Greene/Wiley, New York; Innis, *et al.* (eds.)(1990) PCR Protocols: A *Guide to Methods and Applications* Academic Press, N.Y.

Methods for protein purification include such methods as ammonium sulfate precipitation, column chromatography, electrophoresis, centrifugation, crystallization, and others. See, e.g., Ausubel, *et al.* (1987 and periodic supplements); Deutscher (1990) "Guide to Protein Purification" in *Methods in Enzymology,* vol. 182, and other volumes in this series; and manufacturer's literature on use of protein purification products, e.g., Pharmacia, Piscataway, N.J., or Bio-Rad, Richmond, CA. Combination with recombinant techniques allow fusion to appropriate segments, e.g., to a FLAG sequence or an equivalent which can be fused via a protease-removable sequence. See, e.g., Hochuli (1989) *Chemische Industrie* 12:69-70; Hochuli (1990) "Purification of Recombinant Proteins with Metal Chelate Absorbent" in Setlow (ed.) *Genetic Engineering, Principle and Methods* 12:87-98, Plenum Press, N.Y.; and Crowe, *et al.* (1992) Q/A express: *The High Level Expression* & *Protein Purification System* QUIAGEN, Inc., Chatsworth, CA.

FACS analyses are described in Melamed, *et al.* (1990) *Flow Cytometry and Sorting* Wiley-Liss, Inc., New York, NY: Shapiro (1988) *Practical Flow Cytometry* Liss, New York, MY; and Robinson, *et al.* (1993) *Handbook of Flow Cytometry Methods* Wiley-Liss, New York, NY.

### Production and Sequencing of the Flt3 Ligand

### Assays for the Flt3/Flk2 ligand

Baf3 cells (mouse pre-B cell line; see Palacios, *et al.* (1985) *Cell 41*:727-734; and Palacios, *et al.* (1984) *Nature* 309:126-131) were stably transfected with the cDNA for the Flt3 receptor, Rosnet, *et al.* (1991) *Oncogene 6*:1641-1650. Two methods were used to observe the response of these cells to samples containing the ligand. The main assay is an MTT assay in which the Flt3-transfected cells (Baflts) survive a 24-hour incubation in the presence of the ligand (but not if the ligand is absent) and can then take up and cleave the MTT dye. See Mosmann (1983) *J*. *Immunol Methods 65*:55-63. Untransfected Baf cells die both in the presence and absence of the ligand, and samples are therefore routinely assayed in parallel on both cell types. The difference in signal obtained from these two cell types (Bafft-Baf) is a measure of the amount of ligand present in a sample.

In some cases where active samples also contain substances that are toxic to the cells, the MTT assay with Baftts can fail to detect the presence of the ligand. A second method for detecting the ligand in these cases is to take advantage of the fact that receptors such as c-fms, c-kit, and fit-3 rapidly (within 5 minutes) auto-phosphorylate themselves while binding their appropriate ligands. This response is much less sensitive to toxic substances than is the 24-hour MTT assay.

Auto-phosphorylated receptor is observed by lysing the responder cells with detergent, e.g., NP-40, immunoprecipitating Flt3 using antibodies directed against the intracellular portion of the molecule, and separating the immunoprecipitate on SDS-PAGE, blotting to nitrocellulose, probing with an anti-phosphotyrosine monoclonal antibody, and detecting phosphotyrosine, e.g., with a horseradish peroxidase-linked anti-immunoglobulin and developing with a chemiluminescent substrate.

### Source of the Ftt3 Ligand

Multiple cell lines were screened for onewhich expresses a Flt3ligand. A mouse thymic stromal cell line TA4 (provided by Donna Rennick, DNAX Research Institute) was selected for its ease in handling for large scale production. Cells were grown on roller bottles and 7 day-conditioned media supernatants (serum-free) were harvested, passed through 0.22 µm filters, concentrated 100-fold, and stored frozen at -8D° C.

### Biochemical Characterization of the Flt3 Ligand.

The Flt3 ligand activity has been defined by its separation parameters using different protein separation techniques. However, because of low expression levels, the detection of the activity was greatly facilitated by the concentration of the media. Aliquots of 100x concentrated TA4 cell supernatant, typically representing 10 L of crude supernatant, were subjected to various biochemical purification techniques including ammonium sulfate precipitation, hydrophobic interaction chromatography, anion and cation exchange chromatography, gel filtration chromatography, and reversed phase chromatography. See Table 2. The behavior of the biological activity representing the Fit3 ligand in each of these techniques is summarized below:
Ammonium Sulfate precipitation (at 4° C): activity found in 60-85% saturated (NH₄)₂SO₄ pellet;
Hydrophobic Interaction Chromatography [(NH₄)₂SO₄ gradient in 20 mM Tris, pH 7.5 on a Phenyl-5PW column]: activity eluted between 900-750 mM (NH₄)₂SO₄;
Anion Exchange Chromatography (NaCl gradient in 20 mM Tris, pH 7.5 on Mono Q column): activity eluted between 130-250 mM NaCl;
Cation Exchange Chromatography (NaCl gradient in 10 mM citrate, pH 3.0 on Mono S column): the bulk of the activity eluted between 440-540 mM NaCl;
Gel Filtration (SEPHACRYL® S200 column): the activity ran with an apparent molecular weight of 70 kD;
Reversed Phase HPLC (water to acetonitrile gradient in 0.1% TFA on a Poros R/H column): the activity eluted between 32-35% acetonitrile.

### Engineering. Production and Purification of Soluble Flt3

A soluble fragment of the Flt3 receptor was constructed by removing the membrane spanning and cytoplasmic domains of the Flt3 receptor, fused to a sequence, e.g.. FLAG, useful for purifying the expression product of the construct. See, e.g., Crowe, *et al.* (1992) *QIAexpress: The High Level Expression* & *Protein Purification System* QUIAGEN, Inc. Chatsworth, CA; and Hopp, *et al.* (1988) *Bio*/*Technology 6*:1204-1210. The sequence allows for efficient affinity purification of the soluble product. Appropriate secretion or processing sites may also be engineered into the construct by standard methods. Purification may be achieved by use of affinity purification, e.g., antibodies against the receptor, or by standard protein purification methods. Typically, the affinity reagents or purification procedures can be performed using recombinant receptor.

More specifically, two tags were engineered to the carboxy terminus of the extracellular domain of the Flt3 receptor. pMEXneo-Flt3 was used as a source to modify the Flt3 cDNA to introduce a BgIII site at nucleotide 1662, directly following Ser544, the last amino acid of the extracellular domain. An Xmal/BgIII fragment containing the entire extracellular Flt3 domain was cloned into pHFBgl, a derivative of pVL1393 from Invitrogen Corp. The pHFBgl contains a His₆-FLAG-stop codon sequence fused in frame with the BgIII site of the polylinker. See Table 4. The resulting plasmid was named pHF/FH3.

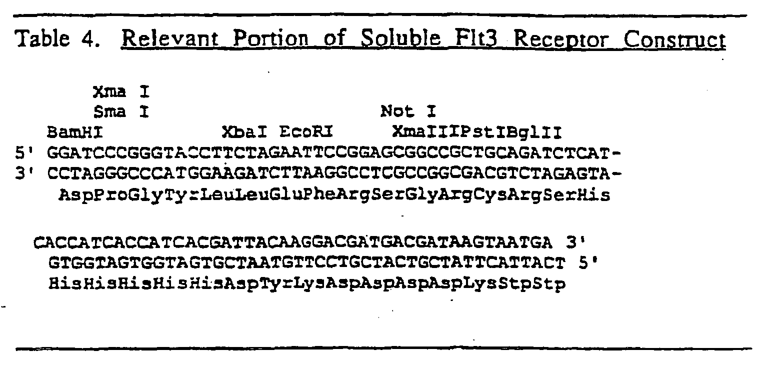

The nucleotide and amino acid sequences shown in Table 4 are also defined in the Sequence Listing by SEQ ID NOs: 36 and 37, respectively.

Sf9 insect cells were transfected with pHF/Fit and a virus stock was prepared. After infection of Sf9 cells with recombinant virus, medium was collected. The soluble secreted Fit3-His₆-FLAG expression product was purified from the medium using a nickel-NTA resin from Qiagen. The purified Flt3 receptor was coupled to an M2 anti-FLAG antibody column. See below.

### Purification of the Fit3 Ligand

The Flt3 ligand was isolated by a combination of affinity chromatography using the Fit3 receptor as a specific binding reagent in combination with the earlier defined physical properties allowing separation from other proteins and contaminants. Similar techniques using human cell assays and human cell sources could be applied to isolate a human ligand.

100L of crude TA4 supernatant was buffer exchanged into PBS and concentrated to 1000x. This was then tumbled overnight with 2 ml M2 (anti-FLAG) beads that had been pre-loaded with soluble Flt3. The beads were then washed with several column volumes of PBS, and the bound material was eluted with 3 ml 100 mM glycine, pH 2.5. The eluate was collected into a tube containing sufficient 2 M Tris, pH 7.5 to neutralize the glycine. The neutralized eluate was then loaded onto a 4.6 x 100 mm Poros R/H column and the proteins chromatographed with a linear water/acetonitrile gradient in 0.1% TFA.

Samples of each fraction were dried down for bioassay and SDS-PAGE. The molecule representing the Flt3 ligand activity ran on reduced SDS-PAGE as an apparently glycosylated protein at approximately 30 kilodaltons. Fractions containing the bulk of the biological activity were dried down completely and combined into sufficient Laemmli gel sample buffer (containing DTT) to be run in a single lane on a 12% mini-gel (SDS-PAGE). The gel was stained with Coomassie blue, destained, and the band representing the Flt3 ligand was carefully excised. This slice contained biochemically pure Flt3 ligand. 100 L of TA4 cell supernatant contained less than 5 micrograms of this protein. The purified protein has a specific activity of 1 x 10⁷ Units per milligram on the Baf assay. One unit is that which provides half maximum stimulation in a 100 µl assay.

### Generation and Purification of Flt3 Ligand Tryptic Peptides

The prep gel slice containing the ligand was briefly rinsed with water and acetonitrile to remove excess SDS, smashed into tiny fragments, taken to dryness under vacuum on a SPEEDVAC® (Savant), and then solubilized in 0.2 ml Tris buffer (pH 7.5) containing 0.5 µg modified trypsin (a chemically modified form that will not digest itself) plus 2 mM DTT, and 0.01% Tween 20. The cleavage reaction was carried out at 37° C for 6 hr, at which time a second 0.5 µg aliquot of trypsin was added, and the digestion continued overnight. The reaction mix-was spun at 13 K and loaded onto a 2.1 x 100 mm AQUAPORE® RP-300 reversed phase column, and peptides eluted with a linear 4-44% acetonitrile gradient (with constant 0.1% TFA). In some cases peptides were rechromatographed on the same column with a 16 - 44% acetonitrile gradient (with constant 0.1 mM heptafluorobutyric acid (HFBA)). Eluting peptides were monitored at both 215 nm and 280 nm and were collected by hand.

### Determination of the Amino Acid Sequence of Peptides of the Flt3 Ligand

Peptide sequences were determined using an Applied Biosystems 477A Sequencer. Fragments provided peptide sequences of Table 1 reconstructed into consensus sequences; peptide 17 is the amino terminus:
12. FVQTXISHLLK
13. DYPVTVAVNLQ
14. TPDAYFSHSPIS:SNFKVKFRELTVHLLK
15. WIEQLK
16. ILFXLFAQYR
17. TPDCY FSHSP ISSNF KVKFR ELTVH LLKDY PVTVA VNLQD EK

### Isolation of a DNA Clone Encoding Flt3 Ligand

The purified protein or defined peptides are useful for generating antibodies by standard methods, as described above. Synthetic peptides or purified protein are presented to an immune system to generate monoclonal or polyclonal antibodies. See, e.g., Coligan (1991) *Current Protocols in immunology* Wiley/Greene; and Harlow and Lane (1989) *Antibodies: A Laboratory Manual* Cold Spring Harbor Press. Alternatively, the Flt3 receptor is used as a specific binding reagent, and advantage can be taken of its specificity of binding, much like an antibody would be used. In either case, the binding reagent is either labeled as described above, e.g., fluorescence or otherwise, or immobilized to a substrate for panning methods.

The binding composition is used for screening of an expression library made from a cell line which expresses a Flt3 ligand. Standard staining techniques are used to detect or sort intracellular or surface expressed ligand, or surface expressing transformed cells are screened by panning. Screening of intracellular expression is performed by various staining or immunofluorescence procedures. See also McMahan, *et al.* (1991) *EMBO J.* 10:2821-2B32..

For example, on day 0, precoat 2-chamber PERMANOX® slides with 1 ml per chamber of fibronectin. 10 ng/ml in PBS, for 30 min at room temperature. Rinse once with PBS. Then plate COS cells at 2-3 x 10⁵ cells per chamber in 1.5 ml of growth media. Incubate overnight at 37° C.

On day 1 for each sample, prepare 0.5 ml of a solution of 66 µg/ml DEAE-dextran, 66 µM chloroquine, and 4 µg DNA in serum free DME. For each set a positive control is prepared, e.g., of huiL-1D-FLAG cDNA at 1 and 1/200 dilution, and a negative mock Rinse cells with serum free DME. Add the DNA solution and incubate 5 hr at 37° C. Remove the medium and add 0.5 ml 10% DMSO in DME for 2.5 min. Remove and wash once with DME. Add 1.5 ml growth medium and incubate overnight.

On day 2, change the medium, On days 3 or 4, the cells are fixed and stained. Rinse the cells twice with Hank's Buffered Saline Solution (HBSS) and fix in 4% paraformaldehyde (PFA)/glucose for 5 min. Wash 3X with HBSS. The slides may be stored at -80° C after all liquid is removed. For each chamber, 0.5 ml incubations are performed as follows. Add HBSS/saponin(0.1%) with 32 µl/ml of 1 M NaN₃ for 20 min. Cells are then washed with HBSS/saponin 1X. Soluble Flt3 receptor/antibody complex to cells and incubate for 30 min. Wash cells twice with HBSS/saponin. Add second antibody, e.g., Vector anti-mouse antibody, at 1/200 dilution, and incubate for 30 min. Prepare ELISA solution, e.g., Vector Elite ABC horseradish peroxidase solution, and preincubate for 30 min. Use, e.g., 1 drop of solution A (avidin) and 1 drop solution B (biotin) per 2.5 ml HBSS/saponin. Wash cells twice with HBSS/saponin. Add ABC HRP solution and incubate for 30 min. Wash cells twice with HBSS, second wash for 2 min, which closes cells. Then add Vector diaminobenzoic acid (DAB) for 5 to 10 min. Use 2 drops of buffer plus 4 drops DAB plus 2 drops of H₂O₂ per 5 ml of glass distilled water. Carefully remove chamber and rinse slide in water. Air dry for a few minutes, then add 1 drop of Crystal Mount and a cover slip. Bake for 5 min at 85-90° C.

Alternatively the binding compositions are used to affinity purify or sort out cells expressing the ligand. See, e.g., Sambrook, *et al.* or Ausubel *et al.*

In another method, the peptide segments are used to predict appropriate oligonucleotides to screen a library. The genetic code is used to select appropriate oligonucleotides useful as probes for screening a library. See, e.g., Table 3. In combination with polymerase chain reaction (PCR) techniques, synthetic oligonucleotides in appropriate orientations are used as primers to select correct clones from a library. Various combinations of upstream/down-stream senselartisense combinations are tested until an appropriate clone is amplified and detected.

For example, a functional screening approach is applied based upon a biological assay for the ligand. The Baf pro-B cell line is transfected with the receptor cDNA to generate a stable transfectant. The transfectant exhibits a mitogenic response to the TA4 stromal cell supernatartts, especially detectable when the supernatant is concentrated 100 X. The parental transfectant cells are non-responsive to the supernatants. An expression cDNA library made from the producing TA4 cells is made. Pools of random cDNA clones are transfected into COS monkey cells and supernatants are screened for ligand biological activity. Positive pools are subdivided to isolate single clones.

In another approach, expression screening is based upon a biochemical assay for a soluble ligand. The assay is based upon the autophosphorylation activity rather than the mitogenic activity. Total cell lysates of the exposed test cells are prepared and immunoprecipitated with anti-phosphotyrosine antiserum. The immunoprecipitate is run on a polyacrylamide gel, and is, e.g., blotted to a filter and developed with antibody staining of phosphotyrosine.

In yet another approach, functional screening of a membrane bound ligand is by either mitogenic bioassay or biochemical autophosphorylation assay. Appropriate fusion vectors can produce an appropriate expression construct

Another strategy is to screen for a membrane bound ligand by panning. The receptor cDNA is constructed as described above. The soluble receptor or antibodies raised against the defined peptide fragments can be immobilized and used to immobilize expressing cells. Immobilization may be achieved by use of appropriate antibodies which recognize, e.g., the FLAG sequence of the soluble receptor construct, or by use of antibodies raised against the first antibodies. Recursive cycles of selection and amplification lead to enrichment of appropriate clones and eventual isolation of ligand expressing clones.

Phage expression libraries can be screened by soluble receptor or anti-fragment antibodies. Appropriate label techniques, e.g., anti-FLAG antibodies, will allow specific labeling of appropriate clones.

Screening by hybridization using degenerate probes based upon the peptide sequences will also allow iso-iation of appropriate clones. Alternatively, use of appropriate primers for PCR screening will yield enrichment of appropriate nucleic acid clones.

Similar methods are applicable to isolate either species or allelic variants. Species variants are isolated using cross-species hybridization techniques based upon isolation of a full length isolate or fragment from one species as a probe. Alternatively, similar assays can be developed, e.g., in human cells for isolation of the Flt3 ligand activity. The mouse cell assays detect a human Flt3 ligand activity, e.g., in human thymic epithelial SV48 cells.

PCR amplification using degenerate primers based upon vector and downstream sequences generated products in a first round of PCR amplification. Primers based upon a vector sequence and degenerate primers from the NLQDEK peptide sequence were used in the first round of amplification. The product of the first amplification was subjected to a second round of PCR amplification using degenerate primers based upon TPDCYF and YPVTVAV peptide sequences. The product of this second round of PCR amplification resulted in a 108 bp product probed using probes based upon NFKVKF sequence. The length is consistent with that predicted by the provided sequence. Sequencing of this 108 bp product provided the nucleotide sequence: which encodes the expected peptide sequence. Further sequencing of a clone has provided the sequence of Table 5.

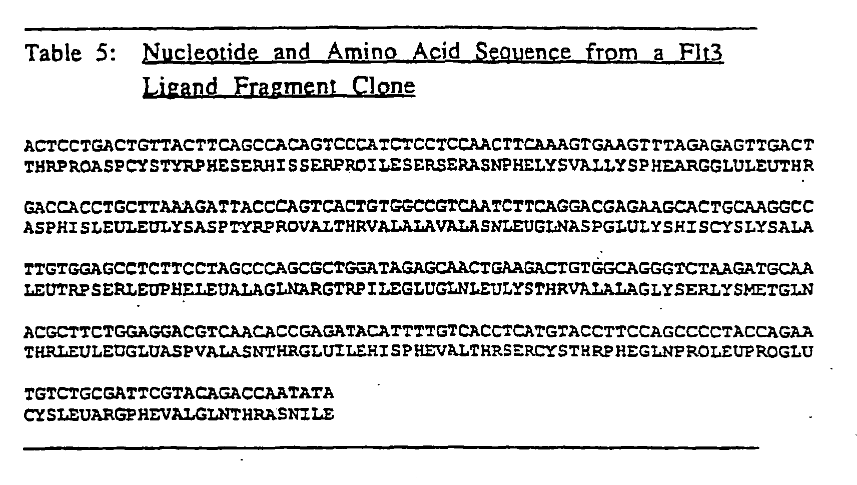

The sequences shown in Table 5, which are also defined in the Sequence Listing by SEQ ID NO: 18, eliminate degeneracy of the relevant encoding nucleic acid sequence. This probe is used to screen longer or full length libraries, eg., from mouse or other mammalian species. Allelic variants and other related genes will also be isolatable using, e.g., hybridization techniques. See Sambrook, *et al.* and Ausubel, *et al.*

### Isolation of a DNA Clone Encoding the Human Flt3 Ligand.

A human 29SV48 stromal cell cDNA library in pME18S, constructed from poly A+ mRNA, was screened with an 800 bp DNA fragment derived from the mouse T118 clone. This fragment encompasses the coding region conserved between the two mouse clones, T118 and T110 (see Table 3). Hybridization of filters with ³²P labeled probe was carried out in a solution of 20% formamide, 6 X SSPE, 5 X Denhardt's, 100 µg/ml tRNA, and 0.1% SDS at 42° C overnight. Filters were washed two times at room temperature with 2 X SSC and 0.1% SDS for 10-15 minutes. Filters were then washed in 0.1 X SSPE, 0.1% SDS under one of the following conditions: three times at 50° C for 30 min each, three times at 55° C for 30 min each; once at 55° C for 30 min and once at 60° C for 30 min; or once at 60° C for 30 min.

Approximately 20 positive colonies were selected using these conditions. These were partially sequenced and two clones, S86 and S109 were found to be approximately 75% homologous to the mouse clones over the first 163 amino acids. Clone S86 continued to show homology to done T110 until the stop codon, although to a lesser degree, for an overall homology of 66%. Clones T118 and S109 do not show homology to each other or the other clones after mouse residue 163 (human residue 160). An additional mouse clone designated MB8 has a 29 amino acid insert at the junction between the common and divergent portions of the mouse ligand.

## Claims

1. An isolated Fms-like tyrosine kinase 3 (Flt3) ligand, comprising the amino acid sequence of SEQ ID No: 19

2. An isolated nucleic acid encoding a Flt3 ligand as claimed in Claim 1.

3. An isolated nucleic acid according to Claim 2 comprising the coding sequence of SEQ ID No: 19.

4. A recombinant vector comprising a nucleic acid of any of Claims 2 to 3.

5. A host cell comprising a recombinant vector of claim 4.

6. A method for making a Flt3 ligand comprising culturing a host cell of Claim 5 under conditions in which the nucleic acid is expressed.

7. The method of Claim 6 in which the Flt3 ligand is isolated from the culture.

8. A pharmaceutical composition comprising a physiologically acceptable carrier and Flt3 ligand of Claim 1.

9. A Flt3 ligand of Claim 1 which has been fused to a polypeptide or labeled with a detectable group.

10. An isolated murine Fms-like tyrosine kinase 3 (Flt3) ligand which specifically binds to a Flt3 tyrosine kinase receptor which Flt3 ligand binds to an antibody produced against a murine Flt3 ligand **characterized by**:
(a) an apparent molecular weight of about 30 Kd in SDS-polyacrylamide gel electrophoresis;
(b) presence in a 60-85% saturated pellet during ammonium sulfate precipitation at 4°C;
(c) elution at between 900-750 mM (NH₄)₂SO₄ in 20 mM Tris buffer, pH 7.5, during gradient hydrophobic interaction chromatography using a phenyl-5PW column;
(d) elution at between 130-250 mM in a NaCl gradient in 20 mM Tris buffer, pH 7.5, during Mono Q column anion exchange chromatography;
(e) elution at between 440-540 mM in a NaCl gradient in 10mM citrate buffer, pH 3.0, during Mono S column cation exchange chromatography;
(f) an apparent molecular weight of 70 kD in SEPHACRYL® S200 gel filtration chromatography; and
(g) elution between 32-35% acetonitrile during reversed phase HPLC using a water acetonitrile gradient in 0.1 % TFA and a Poros R/H column.

11. An antibody or binding fragment thereof which specifically binds to a Flt3 ligand of Claim 10.

12. The antibody of Claim 11 which is monoclonal antibody.

## Revendications

1. Ligand de tyrosine kinase 3 ressemblant à Fms (Fit 3) isolé, comprenant une séquence d'acides aminés définie par SEQ ID No : 19.

2. Acide nucléique isolé codant un ligand de Flt3 selon la revendication 1.

3. Acide nucléique isolé selon la revendication 2, comprenant la séquence codante de SEQ ID No : 19.

4. Vecteur recombinant comprenant un acide nucléique selon l'une des revendications 2 à 3.

5. Cellule hôte comprenant un vecteur recombinant de la revendication 4.

6. Méthode de production d'un ligand de Flt3, comprenant la culture d'une cellule hôte de la revendication 5 dans des conditions dans lesquelles l'acide nucléique est exprimé.

7. Méthode de la revendication 6, dans laquelle le ligand de Flt3 est isolé à partir de la culture.

8. Composition pharmaceutique comprenant un véhicule physiologiquement acceptable et un ligand de Flt3 de la revendication 1.

9. Ligand de Flt3 de la revendication 1 qui a été fusionné à un polypeptide ou marqué par un groupe détectable.

10. Ligand de tyrosine kinase 3 ressemblant à Fms (Flt3) isolé de murin qui se lie spécifiquement à un récepteur de tyrosine kinase Flt3, lequel ligand de Flt3 se lie à un anticorps produit contre un ligand de Flt3 de murin **caractérisé par** :
(a) un poids moléculaire apparent d'environ 30 Kd dans une électrophorèse sur gel de polyacrylamide-SDS ;
(b) une présence dans une boulette saturée à 60-85 % pendant une précipitation en sulfate d'ammonium à 4°C ;
(c) élution avec (NH₄)₂SO₄ entre 900-750 mM dans le tampon Tris 20 mM, pH 7,5, pendant une chromatographie par interaction hydrophobe avec gradient en utilisant une colonne de phényl-5PW
(d) élution entre 130-250 mM dans un gradient de NaCl dans le tampon Tris 20 mM, pH 7,5, pendant une chromatographie par échange d'anions en colonne Mono Q ;
(e) élution entre 440-540 mM dans un gradient de NaCl dans un tampon de citrate 10 mM, pH 3,0, pendant une chromatographie par échange de cations en colonne Mono S ;
(f) un poids moléculaire apparent de 70 Kd dans une chromatographie par filtration sur gel SEPHACRYL® S200 ; et
(g) élution entre 32-35 % d'acétonitrile pendant HPLC en phase inverse en utilisant un gradient d'acétonitrile dans l'eau dans 0,1 % de TFA et une colonne Poros R/H.

11. Anticorps ou son fragment de liaison qui se lie spécifiquement à un ligand de Flt3 selon la revendication 10.

12. Anticorps de la revendication 11 qui est un anticorps monoclonal.

## Patentansprüche

1. Isolierter Ligand der Fms-artigen Tyrosin-Kinase 3 (Flt3), der die Aminosäurensequenz der SEQ ID: 19 umfasst.

2. Isolierte Nukleinsäure, die für einen Flt3-Liganden gemäß Anspruch 1 kodiert.

3. Isolierte Nukleinsäure gemäß Anspruch 2, welche die kodierende Sequenz der SEQ ID NO: 19 umfaßt.

4. Rekombinanter Vektor, der eine Nukleinsäure gemäß einem der Ansprüche 2-3 umfaßt.

5. Wirtszelle, die einen rekombinanten Vektor gemäß Anspruch 4 umfaßt.

6. Verfahren zur Herstellung eines Flt3-Liganden, umfaßend das Kultivieren einer Wirtszelle gemäß Anspruch 5 unter Bedingungen, bei denen die Nukleinsäure exprimiert wird.

7. Verfahren gemäß Anspruch 6, wobei der Flt3-Ligand aus der Kultur isoliert wird.

8. Pharmazeutische Zusammensetzung, die einen physiologisch annehmbaren Träger und einen Flt3-Liganden gemäß Anspruch 1 umfaßt.

9. Flt3-Ligand gemäß Anspruch 1, der mit einem Polypeptid verschmolzen oder mit einer nachweisbaren Gruppe markiert ist.

10. Isolierter Ligand der murinen Fms-artigen Tyrosin-Kinase 3 (Flt3), der spezifisch an einen Flt3-Tyrosin-Kinase-Rezeptor bindet, wobei der Flt3-Ligand spezifisch an einen Antikörper bindet, der gegen einen murinen Flt3-Liganden erzeugt wurde.
(a) ein scheinbares Molekulargewicht von etwa 30 KD bei SDS-Polyacrylamid-Gel-Elektrophorese;
(b) Auftreten in einem Sediment bei 60-85% Sättigung, bei der Ammoniumsulfat-Fällung bei 4°C;
(c) Elution durch 900-750 mM (NH₄)₂SU₄ in 20 mM Tris-Puffer, pH 7,5, während der Gradienten-Hydrophob-Chromatographie unter Verwendung einer Phenyl-5PW-Säule;
(d) Elution durch 130-250 mM in einem NaCl-Gradienten in 20 mM Tris-Puffer pH 7,5, während der Mono-Q-Säulen-Kationenaustausch-Chromatographie;
(e) Elution durch 440-540 mM in einem NaCl-Gradienten in 10 mM Citrat-Puffer, pH 3,0, während der Mono-S-Säulen-Kationenaustausch-Chromatographie;
(f) ein scheinbares Molekulargewicht von 70 KD in SEPH-ACRYL® -S200-Gelfiltrationschromatographie; und
(g) Elution durch 32-35% Acetonitril bei Umkehrphasen-HPLC unter Verwendung eines Wasser-Acetonitril-Gradienten in 0,1% TFA und einer Poros-R/H-Säule.

11. Antikörper oder bindendes Fragment davon, der bzw. das spezifisch an einen Flt3-Liganden gemäß Anspruch 10 bindet.

12. Antikörper gemäß Anspruch 11, bei dem es sich um einen monoklonalen Antikörper handelt.


### SEQUENCE LISTING

(1) GENERAL INFORMATION:
   (i) APPLICANT:
      (A) NAME: Schering Corporation
      (B) STREET: One Giralda Farms
      (C) CITY: Madison
      (D) STATE: New Jersey
      (E) COUNTRY: U.S.A.
      (F) POSTAL CODE (ZIP): 07940-1000
      (G) TELEPHONE: 201-822-7375
      (H) TELEFAX: 201-822-7039
      (I) TELEX: 219165
   (i) APPLICANT:
      (A) NAME: Institut National De La Sante Et De La Recherche Medicale
      (B) STREET: 101 rue de Tolbiac
      (C) CITY: 75643 Paris
      (D) STATE: Cedex 13
      (E) COUNTRY: France
      (F)POSTALCODE(ZIP):
      (G) TELEPHONE: 33 1 44 23 60 41
      (H) TELEFAX: 33 1 45 85 07 66
      (1) TELEX:
   (ii) TITLE OF INVENTION: Purified Mammalian Flt3
      Ligands and Agonists and
      Antagonists Thereof
   (iii) NUMBER OF SEQUENCES: 37
   (iv) COMPUTER READABLE FORM:
      (A) MEDIUM TYPE: Floppy disk
      (B) COMPUTER: Apple Macintosh
      (C) OPERATING SYSTEM: Macintosh 6.0.5
      (D) SOFTWARE: Microsoft Word 5.1a
   (v) CURRENT APPLICATION DATA:
      (A) APPLICATION NUMBER:
      (B) FILING DATE:
   (vi) PRIOR APPLICATION DATA:
      (A) APPLICATION NUMBER: US 08/162,413
      (B) FILING DATE: 03-DEC-1993
   (vi) PRIOR APPLICATION DATA:
      (A) APPLICATION NUMBER: US 08/155,111
      (B) FILING DATE: 19-NOV-1993
   (vi) PRIOR APPLICATION DATA:
      (A) APPLICATION NUMBER: US 08/112,391
      (B) FILING DATE: 24-AUG-1993
   (vi) PRIOR APPLICATION DATA:
      (A) APPLICATION NUMBER: US 08/106,340
      (B) FILING DATE: 13-AUG-1993
   (vi) PRIOR APPLICATION DATA:
      (A) APPLICATION NUMBER: US 08/092,549
      (B) FILING DATE: 16-JUL-1993
   (vi) PRIOR APPLICATION DATA:
      (A) APPLICATION NUMBER: US 08/089,263
      (B) FILING DATE: 07-JUL-1993
   (vi) PRIOR APPLICATION DATA:
      (A) APPLICATION NUMBER: US 08/065,231
      (B) FILING DATE: 19-MAY-1993
(2) INFORMATION FOR SEQ ID NO:1:
   (i) SEQUENCE CHARACTERISTICS:
      (A) LENGTH: 11 amino acids
      (B) TYPE: amino acid
      (D) TOPOLOGY: linear
   (ii) MOLECULE TYPE: peptide
   (xi) SEQUENCE DESCRIPTION: SEQ ID NO:1:
   Phe Val Gln Thr Xaa Ile Ser His Leu Leu Lys
   1               5                   10
(2) INFORMATION FOR SEQ ID NO:2:
   (i) SEQUENCE CHARACTERISTICS:
      (A) LENGTH: 14 amino acids
      (B) TYPE: amino acid
      (D) TOPOLOGY: linear
   (ii) MOLECULE TYPE:peptide
   (xi) SEQUENCE DESCRIPTION: SEQ ID NO:2:
   Asp Tyr Pro Val Thr Val Ala Val Asn Leu Gln Asp Glu Lys
   1               5                   10
(2) INFORMATION FOR SEQ ID NO:3:
   (i) SEQUENCE CHARACTERISTICS:
      (A) LENGTH: 16 amino acids
      (B) TYPE: amino acid
      (D) TOPOLOGY: linear
   (ii) MOLECULE TYPE: peptide
   (xi) SEQUENCE DESCRIPTION: SEQ ID NO:3:
   Thr Pro Asp Xaa Tyr Phe Ser His Ser Pro Ile Ser Ser Asn Phe Lys
   1               5                   10                  15
(2) INFORMATION FOR SEQ ID NO:4:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:4:
   Trp Ile Glu Gln Leu Lys Gln Pro Gly Ser
   1               5                   10
(2) INFORMATION FOR SEQ ID NO:5:
   (i) SEQUENCE CHARACTERISTICS:
      (A) LENGTH: 8 amino acids
      (B) TYPE: amino acid
      (D) TOPOLOGY: linear
   (ii) MOLECULE TYPE: peptide
   (xi) SEQUENCE DESCRIPTION: SEQ ID NO:5:
   Glu Leu Thr Val His Leu Leu Lys
   1 5
(2) INFORMATION FOR SEQ ID NO:6:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:6:
   Ile Leu Phe Xaa Leu Phe Leu Gln Tyr Arg
   1 5 10
(2) INFORMATION FOR SEQ ID NO:7:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:7:
   Ser His Ser Pro Ile Ser Ser Asn Phe
   1 5
(2) INFORMATION FOR SEQ ID NO:8:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (xi) SEQUENCE DESCRIPTION: SEQ ID NO:8:
   Trp Ile Glu Gln Leu Lys
   1 5
(2) INFORMATION FOR SEQ ID NO:9:
   (i) SEQUENCE CHARACTERISTICS:
      (A) LENGTH: 11 amino acids
      (B) TYPE: amino acid
      (D) TOPOLOGY: linear
   (ii) MOLECULE TYPE: peptide
   (xi) SEQUENCE DESCRIPTION: SEQ ID NO:9:
   Asp Tyr Pro Val Thr Val Ala Val Asn Leu Gln
   1               5                   10
(2) INFORMATION FOR SEQ ID NO:10:
   (i) SEQUENCE CHARACTERISTICS:
      (A) LENGTH: 22 amino acids
      (B) TYPE: amino acid
      (D) TOPOLOGY: linear
   (ii) MOLECULE TYPE: peptide
   (xi) SEQUENCE DESCRIPTION: SEQ ID NO:10:
   Asp Ala Tyr Phe Ser His Ser Pro Ile Ser Ser Asn Phe Lys Val Lys
   1               5                   10                  15
   Phe Arg Glu Leu Thr Val
               20
(2) INFORMATION FOR SEQ ID NO:11:
   (i) SEQUENCE CHARACTERISTICS:
      (A) LENGTH: 8 amino acids
      (B) TYPE: amino acid
      (D) TOPOLOGY: linear
   (ii) MOLECULE TYPE: peptide
   (xi) SEQUENCE DESCRIPTION: SEQ ID NO:11:
   Asp Tyr Pro Val Thr Val Ala Ala
   1               5
(2) INFORMATION FOR SEQ ID NO:12:
   (i) SEQUENCE CHARACTERISTICS:
      (A) LENGTH: 28 amino acids
      (B) TYPE: amino acid
      (D) TOPOLOGY: linear
   (ii) MOLECULE TYPE: peptide
   (xi) SEQUENCE DESCRIPTION: SEQ ID NO:12:
   Thr Pro Asp Ala Tyr Phe Ser His Ser Pro Ile Ser Ser Asn Phe Lys
   1               5                   10                  15
   Val Lys Phe Arg Glu Leu Thr Val His Leu Leu Lys
               20                  25
(2) INFORMATION FOR SEQ ID NO:13:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (xi) SEQUENCE DESCRIPTION: SEQ ID NO:13:
   Trp Ile Glu Gln Leu Lys
   1               5
(2) INFORMATION FOR SEQ ID NO:14:
   (i)SEQUENCE CHARACTERISTICS:
      (A) LENGTH: 11 amino acids
      (B) TYPE: amino acid
      (D) TOPOLOGY: linear
   (ii) MOLECULE TYPE: peptide
   (xi) SEQUENCE DESCRIPTION: SEQ ID NO:14:
   Phe Val Gln Thr Xaa Ile Ser His Leu Leu Lys
   1               5                   10
(2) INFORMATION FOR SEQ ID NO:15:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:15:
   Ile Leu Phe Xaa Leu Phe Ala Gln Tyr Arg
   1               5                   10
(2) INFORMATION FOR SEQ ID NO:16:
   (i) SEQUENCE CHARACTERISTICS:
      (A) LENGTH: 11 amino acids
      (B) TYPE: amino acid
      (D) TOPOLOGY: linear
   (ii) MOLECULE TYPE: peptide
   (xi) SEQUENCE DESCRIPTION: SEQ ID NO:16:
   Asp Tyr Pro Val Thr Val Ala Val Asn Leu Gln
   1               5                   10
(2) INFORMATION FOR SEQ ID NO:17:
   (i) SEQUENCE CHARACTERISTICS:
      (A) LENGTH: 42 amino acids
      (B) TYPE: amino acid
      (D) TOPOLOGY: linear
   (ii) MOLECULE TYPE: peptide
   (xi) SEQUENCE DESCRIPTION: SEQ ID NO:17:
   Thr Pro Asp Cys Tyr Phe Ser His Ser Pro Ile Ser Ser Asn Phe Lys
   1               5                   10                  15
   Val Lys Phe Arg Glu Leu Thr Val His Leu Leu Lys Asp Tyr Pro Val
               20                  25                  30
   Thr Val Ala Val Asn Leu Gln Asp Glu Lys
           35                  40
(2) INFORMATION FOR SEQ ID NO:18:
   (i) SEQLTENCE CHARACTERISTICS:
      (A) LENGTH: 303 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 18:
   ACT CCT GAC TGT TAC TTC AGC CAC AGT CCC ATC TCC TCC AAC TTC AAA    48
   Thr Pro Asp Cys Tyr Phe Ser His Ser Pro Ile Ser Ser Asn Phe Lys
    1               5                  10                  15
   GTG AAG TTT AGA GAG TTG ACT GAC CAC CTG CTT AAA GAT TAC CCA GTC    96
   Val Lys Phe Arg Glu Leu Thr Asp His Leu Leu Lys Asp Tyr Pro Val
               20                  25                  30
   ACT GTG GCC GTC AAT CTT CAG GAC GAG AAG CAC TGC AAG GCC TTG TGG   144
   Thr Val Ala Val Asn Leu Gln Asp Glu Lys His Cys Lys Ala Leu Trp
           35                  40                  45
   AGC CTC TTC CTA GCC CAG CGC TGG ATA GAG CAA CTG AAG ACT GTG GCA   192
   Ser Leu Phe Leu Ala Gln Arg Trp Ile Glu Gln Leu Lys Thr Val Ala
       50                  55                  60
   GGG TCT AAG ATG CAA ACG CTT CTG GAG GAC GTC AAC ACC GAG ATA CAT   240
   Gly Ser Lys Met Gln Thr Leu Leu Glu Asp Val Asn Thr Glu Ile His
   65                  70                  75                  80
   TTT GTC ACC TCA TGT ACC TTC CAG CCC CTA CCA GAA TGT CTG CGA TTC   288
   Phe Val Thr Ser Cys Thr Phe Gln Pro Leu Pro Glu Cys Leu Arg Phe
                   85                  90                  95
   GTA CAG ACC AAT ATA                                               303
   Val Gln Thr Asn Ile
               100
(2) INFORMATION FOR SEQ ID NO:19:
   (i) SEQUENCE CHARACTERISTICS:
      (A) LENGTH: 857 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:19:
   GAAAGGGCTG TCACCCGGCT TGGCCCCTTC CACACCCAAC TGGGGCAAGC CTGACCCGGC  60
   GACAGGAGGC ATGAGGGGCC CCCGGCCGAA ATG ACA GTG CTG GCG CCA GCC TGG  114
                                    Met Thr Val Leu Ala Pro Ala Trp
                                     1               5
   AGC CCA ACA ACC TAT CTC CTC CTG CTG CTG CTG CTG AGC TCG GGA CTC   162
   Ser Pro Thr Thr Tyr Leu Leu Leu Leu Leu Leu Leu Ser Ser Gly Leu
       10                  15                  20
   AGT GGG ACC CAG GAC TGC TCC TTC CAA CAC AGC CCC ATC TCC TCC GAC    210
   Ser Gly Thr Gln Asp Cys Ser Phe Gln His Ser Pro Ile Ser Ser Asp
    25                  30                  35                  40
   TTC GCT GTC AAA ATC CGT GAG CTG TCT GAC TAC CTG CTT CAA GAT TAC    258
   Phe Ala Val Lys Ile Arg Glu Leu Ser Asp Tyr Leu Leu Gln Asp Tyr
                    45                  50                  55
   CCA GTC ACC GTG GCC TCC AAC CTG CAG GAC GAG GAG CTC TGC GGG GCG    306
   Pro Val Thr Val Ala Ser Asn Leu Gln Asp Glu Glu Leu Cys Gly Ala
                60                  65                  70
   CTC TGG CGG CTG GTC CTG GCA CAG CGC TGG ATG GAG CGG CTC AAG ACT    354
   Leu Trp Arg Leu Val Leu Ala Gln Arg Trp Met Glu Arg Leu Lys Thr
            75                  80                  85
   GTC GCT GGG TCC AAG ATG CAA GGC TTG CTG GAG CGC GTG AAC ACG GAG    402
   Val Ala Gly Ser Lys Met Gln Gly Leu Leu Glu Arg Val Asn Thr Glu
        90                  95                 100
   ATA CAC TTT GTC ACC AAA TGT GCC TTT CAG CCC CCC CCC AGC TGT CTT    450
   Ile His Phe Val Thr Lys Cys Ala Phe Gln Pro Pro Pro Ser Cys Leu
   105                 110                 115                 120
   CGC TTC GTC CAG ACC AAC ATC TCC CGC CTC CTG CAG GAG ACC TCC GAG    498
   Arg Phe Val Gln Thr Asn Ile Ser Arg Leu Leu Gln Glu Thr Ser Glu
                   125                 130                 135
   CAG CTG GTG GCG CTG AAG CCC TGG ATC ACT CGC CAG AAC TTC TCC CGG    546
   Gln Leu Val Ala Leu Lys Pro Trp Ile Thr Arg Gln Asn Phe Ser Arg
               140                 145                 150
   TGC CTG GAG CTG CAG TGT CAG CCC GAC TCC TCA ACC CTG CCA CCC CCA    594
   Cys Leu Glu Leu Gln Cys Gln Pro Asp Ser Ser Thr Leu Pro Pro Pro
           155                 160                 165
   TGG AGT CCC CGG CCC CTG GAG GCC ACA GCC CCG ACA GCC CCG CAG CCC    642
   Trp Ser Pro Arg Pro Leu Glu Ala Thr Ala Pro Thr Ala Pro Gln Pro
       170                 175                 180
   CCT CTG CTC CTC CTA CTG CTG CTG CCC GTG GGC CTC CTG CTG CTG GCC    690
   Pro Leu Leu Leu Leu Leu Leu Leu Pro Val Gly Leu Leu Leu Leu Ala
   185                 190                 195                 200
   GCT GCC TGG TGC CTG CAC TGG CAG AGG ACG CGG CGG AGG ACA CCC CGC    738
   Ala Ala Trp Cys Leu His Trp Gln Arg Thr Arg Arg Arg Thr Pro Arg
                   205                 210                 215
   CCT GGG GAG CAG GTG CCC CCC GTC CCC AGT CCC CAG GAC CTG CTG CTT    786
   Pro Gly Glu Gln Val Pro Pro Val Pro Ser Pro Gln Asp Leu Leu Leu
               220                 225                 230
   GTG GAG CAC TGACCTGGCC AAGGCCTCAT CCTGGGGAGG ATACGTAGGC            835
   Val Glu His
           235
   ACACAGAGGG GAGTCACCAG CC                                            857
(2) INFORMATION FOR SEQ ID NO:20:
   (i) SEQUENCE CHARACTERISTICS:
      (A) LENGTH: 301 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:20:
   GGT GCC CCC CGT CCC CAG TCC CCA GGA CCT GCT GCT TGT GGA GCA CTG      48
   Gly Ala Pro Arg Pro Gln Ser Pro Gly Pro Ala Ala Cys Gly Ala Leu
     1               5                  10                  15
   ACC TGG CCA AGG CCT CAT CCT GGG GAG GAT ACT GAG GCA CAC AGA GGG      96
   Thr Trp Pro Arg Pro His Pro Gly Glu Asp Thr Glu Ala His Arg Gly
               20                  25                  30
   GAG TCA CCA GCC AGA GGA TGC ATA GCC TGG ACA CAG AGG AAG TTG GCT     144
   Glu Ser Pro Ala Arg Gly Cys Ile Ala Trp Thr Gln Arg Lys Leu Ala
           35                  40                  45
   AGA GGC CGG TCC CTT CCT TGG GCC CCT CTC ATT CCC TCC CCA GAA TGG     192
   Arg Gly Arg Ser Leu Pro Trp Ala Pro Leu Ile Pro Ser Pro Glu Trp
       50                  55                  60
   AGG CAA CGC CAG AAT CCA GCA CCG GCC CCA TTT ACC CAA CTC TGT ACA     240
   Arg Gln Arg Gln Asn Pro Ala Pro Ala Pro Phe Thr Gln Leu Cys Thr
    65                  70                  75                  80
   AAG CCC TTG TCC CCA TGAAATTGTA TATAAATCAT CCTTTTCTAC CAAAAAAAAA     295
   Lys Pro Leu Ser Pro
                    85
   AAAAAA                                                             301
(2) INFORMATION FOR SEQ ID NO:21:
   (i) SEQUENCE CHARACTERISTICS:
      (A) LENGTH: 1017 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:21:
   GAATTCGCGG CCGCGTCGAG CCTGGCGGGA CTGAGCCCGA GACCTGCCCT CCTGTCACTT    60
   CCAAGAACCT GTCACAGGCA TGAGGGGTCC CCGGCAGAG ATG ACA GTG CTG GCG     114
                                              Met Thr Val Leu Ala
                                                1               5
   CCA GCC TGG AGC CCA AAT TCC TCC CTG TTG CTG CTG TTG CTG CTG CTG     162
   Pro Ala Trp Ser Pro Asn Ser Ser Leu Leu Leu Leu Leu Leu Leu Leu
                    10                  15                  20
   AGT CCT TGC CTG CGG GGG ACA CCT GAC TGT TAC TTC AGC CAC AGT CCC     210
   Ser Pro Cys Leu Arg Gly Thr Pro Asp Cys Tyr Phe Ser His Ser Pro
                25                  30                  35
   ATC TCC TCC AAC TTC AAA GTG AAG TTT AGA GAG TTG ACT GAC CAC CTG     258
   Ile Ser Ser Asn Phe Lys Val Lys Phe Arg Glu Leu Thr Asp His Leu
            40                  45                  50
   CTT AAA GAT TAC CCA GTC ACT GTG GCC GTC AAT CTT CAG GAC GAG AAG     306
   Leu Lys Asp Tyr Pro Val Thr Val Ala Val Asn Leu Gln Asp Glu Lys
        55                  60                  65
   CAC TGC AAG GCC TTG TGG AGC CTC TTC CTA GCC CAG CGC TGG ATA GAG     354
   His Cys Lys Ala Leu Trp Ser Leu Phe Leu Ala Gln Arg Trp Ile Glu
    70                  75                  80                  85
   CAA CTG AAG ACT GTG GCA GGG TCT AAG ATG CAA ACG CTT CTG GAG GAC     402
   Gln Leu Lys Thr Val Ala Gly Ser Lys Met Gln Thr Leu Leu Glu Asp
                    90                  95                 100
   GTC AAC ACC GAG ATA CAT TTT GTC ACC TCA TGT ACC TTC CAG CCC CTA     450
   Val Asn Thr Glu Ile His Phe Val Thr Ser Cys Thr Phe Gln Pro Leu
               105                 110                 115
   CCA GAA TGT CTG CGA TTC GTC CAG ACC AAC ATC TCC CAC CTC CTG AAG     498
   Pro Glu Cys Leu Arg Phe Val Gln Thr Asn Ile Ser His Leu Leu Lys
           120                 125                 130
   GAC ACC TGC ACA CAG CTG CTT GCT CTG AAG CCC TGT ATC GGG AAG GCC     546
   Asp Thr Cys Thr Gln Leu Leu Ala Leu Lys Pro Cys Ile Gly Lys Ala
       135                 140                 145
   TGC CAG AAT TTC TCT CGG TGC CTG GAG GTG CAG TGC CAG CCG GAC TCC     594
   Cys Gln Asn Phe Ser Arg Cys Leu Glu Val Gln Cys Gln Pro Asp Ser
   150                 155                 160                 165
   TCC ACC CTG CTG CCC CCA AGG AGT CCC ATA GCC CTA GAA GCC ACG GAG     642
   Ser Thr Leu Leu Pro Pro Arg Ser Pro Ile Ala Leu Glu Ala Thr Glu
                   170                 175                 180
   CTC CCA GAG CCT CGG CCC AGG CAG CTG TTG CTC CTG CTG CTG CTG CTG     690
   Leu Pro Glu Pro Arg Pro Arg Gln Leu Leu Leu Leu Leu Leu Leu Leu
               185                 190                 195
   CTG CCT CTC ACA CTG GTG CTG CTG GCA GCC GCC TGG GGC CTT CGC TGG     738
   Leu Pro Leu Thr Leu Val Leu Leu Ala Ala Ala Trp Gly Leu Arg Trp
           200                 205                 210
   CAA AGG GCA AGA AGG AGG GGG GAG CTC CAC CCT GGG GTG CCC CTC CCC     786
   Gln Arg Ala Arg Arg Arg Gly Glu Leu His Pro Gly Val Pro Leu Pro
       215                 220                 225
   TCC CAT CCC TAGGATGCGA GCCTTGTGCA TCGTTGACTC AGCCAGGGTC             835
   Ser His Pro
   230
   TTATCTCGAG TTGGGAACCA AAACAAGGAA CAAGCTAGGC AAGTGCTGTG CTGAGTTACA  895
   TCCCCAGCCC AGAGGACACA CTGTCTGGGT ATGGCGATGG ACACTGTAAT TCCAGTGCTT  955
   CTGGATTGGA CATGCTGAAA CTGGATACTG ACTTTAAGAA AAACAGAAAG GAAGAACCCC 1015
   CC                                                                1017
(2) INFORMATION FOR SEQ ID NO:22:
   (i) SEQUENCE CHARACTERISTICS:
      (A) LENGTH: 57 amino acids
      (B) TYPE: amino acid
      (D) TOPOLOGY: linear
   (ii) MOLECULE TYPE: peptide
   (xi) SEQUENCE DESCRIPTION: SEQ ID NO:22:
   Gly Asn Gly Gly Pro Arg Ala Gln His His Gly Ala Thr Arg Leu Thr
   1               5                   10                  15
   Ala Thr Ala Leu Leu Thr Val Cys Pro Gly Leu Leu Leu Pro Leu Val
               20                  25                  30
   Gly Thr Ser His Met Phe Phe Leu Pro Tyr Phe Leu Ser Phe Leu Ser
           35                  40                  45
   Ser Phe Leu Lys Met Tyr Leu Tyr Val
       50                  55
(2) INFORMATION FOR SEQ ID NO:23:
   (i) SEQUENCE CHARACTERISTICS:
      (A) LENGTH: 87 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:23:
   GAT AGG GTC TCA TTA TTA TGC AGG CTA GGC CTG ACC CTG AAC TCA AAG      48
   Asp Arg Val Ser Leu Leu Cys Arg Leu Gly Leu Thr Leu Asn Ser Lys
     1               5                  10                  15
   CAA TCC TCC TGC CTC AGT GTC CTG AGT GCT GGG ATT ACA                  87
   Gln Ser Ser Cys Leu Ser Val Leu Ser Ala Gly Ile Thr
               20                  25
(2) INFORMATION FOR SEQ ID NO:24:
   (i) SEQUENCE CHARACTERISTICS:
      (A) LENGTH: 344 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:24:
   GGTAACGGTG GCCCCAGAGC CCAGCACCAT GGTGCCACCA GGCTCACAGC CACAGCCTTG   60
   CTAACTGTGT GTCCAGGGCT TCTGCTCCCA CTAGTTGGCA CTTCACACAT GTTCTTTCTC  120
   CCTTATTTTC TCTCTTTTCT TTCTTCTTTT TTAAAGATGT ATCTTTATGT GTGAGTGTTT  180
   TACCTACATG CCTGTAAGTG CACTGAATGT GTGTCTGGTG CCTGCAGAGG CCAGAAGAGG  240
   GCACCAGATC CCCTGAAACT GGAGTCTCTN NGCTCCGTGT GAACCACCAC GTGGTGCTGG  300
   GACCCAGGTC CAATGCAAGA GCACCCAGGG TTCTTACCTG CTGA                   344
(2) INFORMATION FOR SEQ ID NO:25:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii)MOLECULE TYPE: cDNA
   (xi) SEQUENCE DESCRIPTION: SEQ ID NO:25:
      TTYGTNCARA CNAAYATH 18
(2) INFORMATION FOR SEQ ID NO:26:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:26:
      TTYGTNCARA CNTGYATH 18
(2) INFORMATION FOR SEQ ID NO:27:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:27:
      TTYGTNCARA CNAGYATH 18
(2) INFORMATION FOR SEQ ID NO:28:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:28:
      TTYGTNCARA CNTCNATH 18
(2) INFORMATION FOR SEQ ID NO:29:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE:cDNA
   (xi) SEQUENCE DESCRIPTION: SEQ ID NO:29:
      TTTYGTNCARA CNACNATH 18
(2) INFORMATION FOR SEQ ID NO:30:
   (i)SEQUENCE CHARACTERISTICS:
      (A) LENGTH: 18 base pairs
      (B) TYPE: nucleic acid
      (C) STRANDEDNESS: single
      (D) TOPOLOGY: linear
         (ii) MOLECULE TYPE: cDNA
         (xi) SEQUENCE DESCRIPTION: SEQ ID NO:30:
            GAYTAYCCNG TNACNGTN 18
(2) INFORMATION FOR SEQ ID NO:31:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:31:
      ACNCCNGAYA TNTAYTTY
      18
(2) INFORMATION FOR SEQ ID NO:32:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:32:
      TBGATHGARC ARCTNAAR 18
(2) INFORMATION FOR SEQ ID NO:33:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:33:
      TGGATHGARC ARTTRAAR 18
(2) INFORMATION FOR SEQ ID NO:34:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:34:
      AAYTTYAARG TNAARTTY 18
(2) INFORMATION FOR SEQ ID NO:35:
   (i) SEQUENCE CHARACTERISTICS:
      (A) LENGTH: 87 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:35:
   ACTCCTGACT GTTACTTCAG CCACAGTCCC ATCTCCTCCA ACTTCAAAGT GAAGTTTAGA 60
   GAGTTGACTG ACCACCTGCT TAAAGAT 87
(2) INFORMATION FOR SEQ ID NO:36:
   (i) SEQUENCE CHARACTERISTICS:
      (A) LENGTH: 94 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:36:
   GGATCCCGGG TACCTTCTAG AATTCCGGAG CGGCCGCTGC AGATCTCATC ACCATCACCA 60
   TCACGATTAC AAGGACGATG ACGATAAGTA ATGA 94
(2) INFORMATION FOR SEQ ID NO:37:
   (i) SEQUENCE CHARACTERISTICS:
      (A) LENGTH: 29 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:37:
   Asp Pro Gly Tyr Leu Leu Glu Phe Arg Ser Gly Arg Cys Arg Ser His
   1               5                   10                  15
   His His His His His Asp Tyr Lys Asp Asp Asp Asp Lys
               20                  25